# EUROPEAN PATENT SPECIFICATION

(11) **EP 2 125 021 B1**
(45) Date of publication and mention of the grant of the patent: **08.06.2011**
(21) Application number: 07857036.3
(22) Date of filing: 21.12.2007
(51) Int. Cl.: A61K 45/06, A61P 13/10

(54) **COMBINATION THERAPY OF LOWER URINARY TRACT DISORDERS WITH 2 LIGANDS AND NSAIDS**
KOMBINATIONSTHERAPIE FÜR ERKRANKUNGEN DER UNTEREN HARNWEGE MIT 2 LIGANDEN UND NSAR
THÉRAPIE COMBINÉE DE TROUBLES DU TRACTUS URINAIRE INFÉRIEUR À L'AIDE DE LIGANDS DE L' <SB>2</SB> ET D'AINS

(30) Priority: 22.12.2006 US 871719 P
(43) Date of publication of application: 02.12.2009
(73) Proprietor: Recordati Ireland Limited, Ringaskiddy County Cork (IE)
(72) Inventor: LEONARDI, Amedeo, 20154 Milano (IT); GUARNERI, Luciano, 20024 Garbagnate Milanese (IT); ANGELICO, Patrizia, 20099 Sesto San Giovanni (IT)
(74) Representative: Marshall, John Grahame
(86) International application number: PCT/EP2007/011313
(87) International publication number: WO 2008/077599

(56) References cited:
- WO-A-99/12537
- WO-A-2004/037190
- WO-A-2006/090275
- US-B1- 6 451 857

## Description

### Field of the Invention

The invention relates to the use of α₂δ calcium channel subunit ligands and non-steroidal anti-inflammatory drugs in the combination treatment of lower urinary tract disorders.

### Background of the Invention

Lower urinary tract disorders encompass an assortment of syndromes that affect normal micturition. Lower urinary tract disorders may develop through combination of pathological and/or age-related changes of the urogenital system, or other etiology, e.g., neurological disorders. Individuals suffering from lower urinary tract disorders suffer from impaired quality of life, including embarrassment, poor self-perception, and a general reduction in emotional well-being, social function, and general health. Lower urinary tract disorders, moreover, may be associated with other physical ailments, including cellulitis, pressure ulcers, urinary tract infections, falls with fractures, sleep deprivation, social withdrawal, depression, and sexual dysfunction. Older individuals suffering from lower urinary tract disorders may require more care from care providers, both family and profession, which may be a factor in decisions to place them in institutions.

According to the U.S. National Institutes of Health (NIH), up to 35 million Americans are estimated to suffer lower urinary tract disorders. Lower urinary tract disorders are more common among women than men (2:1) until age 80, after which men and women are equally affected. The prevalence of lower urinary tract disorders increases with age. By the age 65, lower urinary tract disorders affect 15% to 30% of all individuals and approximately 50% of individuals in long-term care.

Agents with various modes of action have been used to treat lower urinary tract disorders. These include agents that act directly on the lower urinary tract, e.g., antimuscarinics and alpha 1 antagonists, and agents that act through the central nervous system, e.g., serotonin and/or noradrenaline reuptake inhibitors. According to the NIH, however, while some progress has been made in the diagnosis, management, and treatment of lower urinary tract disorders, these disorders frequently remain intractable. Thus, there is a continued need for improved agents, formulations and therapies to treat lower urinary tract disorders.
US 6451857 and WO 99/12537 describe the combination of an anti-epileptic drug such as gabapentin or pregbalin with an NSAID such as celecoxib, diclofenac, diflunisal, naproxen or sulindac for use in the treatment of pain. There is no disclosure that such a combination might be useful in the treatment of lower urinary tract disorders.
WO 2006/090275 describes the use of a synaptic vesicle protein 2A (SV2A) for the treatment of lower urinary tract dysfunction. It refers to use of the SV2A in combination with other drugs, including gabapentin, pregbalin and celecoxib. Disclosure that an SV2A can be used in combination with other drugs does not, however, disclose that combinations of those other drugs can be used, without the SV2A, for the same or any other treatment.
WO 2004/037190 describes a delayed release formulation of milnacipran, a serotonin and norepinephrine reuptake inhibitor used for the treatment of depression. The formulation may also comprise other drugs, including gabapentin, pregbalin and celecoxib. It is alleged that the formulation may be suitable for providing relief from a range of disorders, but lower urinary tract disorders are mentioned in this range. Furthermore, disclosure that milnacipran can be used in combination with other drugs does not, however, disclose that combinations of those other drugs can be used, without milnacipran, for the same or any other treatment.

### Summary of the Invention

The present inventors have unexpectedly found that administration to a mammal of a combination of compounds, at least one of which is an α₂δ calcium channel subunit (A2d) ligand and at least one of which is a non-steroidal anti-inflammatory drug (NSAID), provides a surprising and potent inhibition of the micturition reflex, superior to that obtained by treatment with an A2d ligand or NSAID alone. Combinations of A2d ligand and NSAIDs are thus useful for treatment of lower urinary tract disorders and symptoms thereof.

In one aspect, the invention provides an A2d ligand for use in the treatment of urinary incontinence in a subject suffering from a lower urinary tract disorder in combination with the prior, concurrent or post-administration of an NSAID.

In a second aspect, the invention provides an NSAID for use in the treatment of urinary incontinence in a subject suffering from a lower urinary tract disorder in combination with the prior, concurrent or post-administration of an A2d ligand.

In a third aspect, the invention provides an A2d ligand and an NSAID for use concurrently or sequentially in the treatment of urinary incontinence in a subject suffering from a lower urinary tract disorder.

In a fourth aspect, the invention provides the use of an A2d ligand for the preparation of a medicament for the treatment of urinary incontinence in a subject suffering from a lower urinary tract disorder in combination with the prior, concurrent or post-administration of an NSAID.

In a fifth aspect, the invention provides the use of an NSAID for the preparation of a medicament for the treatment of urinary incontinence in a subject suffering from a lower urinary tract disorder in combination with the prior, concurrent or post-administration of an A2d ligand.

The invention is applicable to lower urinary tract disorders in general, but in particular to overactive bladder (OAB), interstitial cystitis, prostatitis, prostadynia and benign prostatic hyperplasia (BPH). The urinary incontinence may be caused by or associated with such disorders, and may be urge incontinence, stress incontinence, mixed incontinence or overflow incontinence.

In the invention, the A2d ligand is gabapentin, pregabalin, (1R,SR,6S)-6-aminomethyl-6-carboxymethyl-bicyclo[3.2.0]heptane, 3-(1-aminomethyl-cyclohexylmethyl)-4H-1,2,4-oxadiazol-5-one, 5-(1-aminomethyl-cyclohexylmethyl)-1H-tetrazole, (3S,4S)-(1-aminomethyl-1-carboxymethyl-3,4-dimethyl-cyclopentane, (1α,3α,5α)-(3-aminomethyl-3-carboxymethyl-bicyclo[3.2.0]heptane, (3S,5R)-3-aminomethyl-5-methyl-octanoic acid, (3S,5R)-3-amino-5-methyl-heptanoic acid, (3S,5R)-3-amino-5-methyl-octanoic acid, (3S,5R)-3-amino-5-methyl-nonanoic acid, (2S,4S)-4-(3-chlorophenoxy)-proline or (2S,4S)-4-(3-fluorobenzyl)-proline, a pharmaceutically acceptable salt of the foregoing, or a combination thereof.

In preferred embodiments of the invention, an NSAID for treatment of a lower urinary tract disorder or symptom of such a disorder is a selective COX-2 inhibitor. In more specific embodiments, an NSAID for treatment of a lower urinary tract disorder or symptom of such a disorder is celecoxib, diclofenac, diflunisal, flurbiprofen, naproxen, nimesulide or sulindac, a pharmaceutically acceptable salt of the foregoing, or a combination thereof.

An advantage of combination therapy is that unit dosage amounts of each of the active substances (in casu, the A2d ligand and the NSAID) may be less that the amounts required for monotherapy. Side effects of the active substances may be avoided by using such lower doses, without loss of therapeutic effect. This applies in the various embodiments of the invention as set out above. There may indeed be a synergistic effect between the A2d ligand and the NSAID as used in the treatment of urinary incontinence in a subject suffering from a lower urinary tract disorder.

### Detailed Description of the Invention

Combination therapy with an A2d ligand and an NSAID may be used as primary therapy for treatment of an individual suffering from a lower urinary tract disorder, as set forth below or as follow-up, secondary therapy. Thus, lower urinary tract disorders often respond to certain classes or subclasses of therapeutic agents. Furthermore, patients may respond initially to a therapeutic agent, but become non-responsive to the agent overtime. Additionally, patients may exhibit undesirable side effects when therapeutic agents are administered in concentrations required to treat lower urinary tract conditions. Side effects NSAIDs that are non-selective COX inhibitors include gastric ulceration and intolerance, inhibition of platelet function and hypersensitivity. Both non-selective and selective COX-2 inhibitors may alter renal function. The A2d ligand gabapentin may cause side effects of somnolence, dizziness, ataxia, and fatigue. These side effects may be overcome by administering lower dosages of two or more therapeutic agents to achieve a therapeutic effect. The synergistic (i.e., superadditive) effect observed for combination treatment with A2d ligand and NSAID may afford effective treatment of lower urinary tract disorders wherein one or even all of the lower dosages would not be sufficient to have a therapeutic effect when the respective therapeutic agent is used in monotherapy.

The nomenclature of lower urinary tract symptoms and pathologies used herein is set forth in Abrams et al., Neurol. and Urodyn. 21:167-178 (2002) and Andersson et al., Pharmacol. Rev. 56:581-631 (2004).

Voiding dysfunctions can be roughly classified as disturbances of storage or emptying. Storage symptoms are experienced during the storage phase of the bladder, and include increased daytime frequency, nocturia (the waking at night one or more times to void), urgency (a sudden, compelling desire to pass urine that is difficult to defer), and urinary incontinence (the any involuntary leakage of urine). Urinary incontinence may be further characterized according to symptoms. Stress urinary incontinence is the involuntary leakage on effort or exertion, or on sneezing or coughing. Urge urinary incontinence is the involuntary leakage of urine accompanied by or immediately preceded by urgency. Mixed urinary incontinence is the involuntary leakage of urine associated with urgency and also with exertion, effort, sneezing or coughing. Overflow incontinence is the involuntary leakage of urine occurring after the bladder capacity has been exceeded, e.g., from a failure to empty. Enuresis also refers to any involuntary loss of urine. Nocturnal enuresis is the loss of urine occurring during sleep.

Voiding symptoms include slow stream, splitting or spraying of the urine stream, intermittent stream (intermittency, i.e., the stopping and restarting of urine flow during micturition, hesitancy (difficulty in initiating micturition resulting in a delay in the onset of voiding after the individual is ready to pass urine), straining and terminal dribble (a prolonged final part of micturition, when the flow has slowed to a trickle/dribble).

Lower urinary tract disorders may further be categorized by a constellation of symptoms (i.e., a syndrome) or by etiology. Individuals suffering from overactive bladder (OAB) syndrome, e.g., typically suffer from symptoms of urgency, urge incontinence, increased daytime frequency or nocturia. OAB occurs as a result of detrusor muscle overactivity referred to as detrusor muscle instability. Detrusor muscle instability can arise from non-neurological abnormalities, such as bladder stones, muscle disease, urinary tract infection or drug side effects or can be idiopathic.

Neurogenic overactive bladder (or neurogenic bladder) is a type of overactive bladder which occurs as a result of detrusor muscle overactivity referred to as detrusor hyperreflexia, secondary to known neurological disorders. Patients with neurological disorders, such as stroke, Parkinson's disease, diabetes, multiple sclerosis, peripheral neuropathy, or spinal cord lesions often suffer from neurogenic overactive bladder.

Cystitis (including interstitial cystitis) is a lower urinary tract disorder of unknown etiology that predominantly affects young and middle-aged females, although men and children can also be affected. Symptoms of interstitial cystitis can include voiding symptoms, increased daytime frequency, urgency, nocturia or suprapubic or pelvic pain related to and relieved by voiding. Many interstitial cystitis patients also experience headaches as well as gastrointestinal and skin problems. In some cases, interstitial cystitis can also be associated with ulcers or scars of the bladder.

Prostatitis and prostadynia are other lower urinary tract disorders that have been suggested to affect approximately 2-9% of the adult male population. Prostatitis is an inflammation of the prostate, and includes bacterial prostatitis (acute and chronic) and non-bacterial prostatitis. Acute and chronic bacterial prostatitis are characterized by inflammation of the prostate and bacterial infection of the prostate gland, usually associated with symptoms of pain, increased daytime frequency and/or urgency. Chronic bacterial prostatitis is distinguished from acute bacterial prostatitis based on the recurrent nature of the disorder. Chronic non-bacterial prostatitis is characterized by inflammation of the prostate which is of unknown etiology accompanied by the presence of an excessive amount of inflammatory cells in prostatic secretions not currently associated with bacterial infection of the prostate gland, and usually associated with symptoms of pain, increased daytime frequency and/or urgency. Prostadynia is a disorder which mimics the symptoms of prostatitis absent inflammation of the prostate, bacterial infection of the prostate and elevated levels inflammatory cells in prostatic secretions. Prostadynia can be associated with symptoms of pain, increased daytime frequency and/or urgency.

Benign prostatic hyperplasia (BPH) is a non-malignant enlargement of the prostate that is very common in men over 40 years of age. BPH is thought to be due to excessive cellular growth of both glandular and stromal elements of the prostate. Symptoms of BPH can include increased frequency, urgency, urge incontinence, nocturia, and voiding symptoms, including slow stream, splitting or spraying of the urine stream, intermittency, hesitancy, straining and terminal dribble.

In certain aspects the present invention provides the use of an effective amount of a combination of compounds, at least one of which is an A2d ligand and at least one of which is an NSAID, for treating lower urinary tract disorders in a patient in need of such treatment. Treatment of lower urinary tract disorders includes treatment of storage symptoms or voiding symptoms. Treatment of lower urinary tract disorders also includes treatment of increased daytime frequency, nocturia, urgency, urinary incontinence, including urge incontinence, stress incontinence, mixed incontinence and overflow incontinence, enuresis, including nocturnal enuresis, slow stream, splitting or spraying of the urine stream, intermittency, hesitancy, straining and terminal dribble.

Treatment of lower urinary tract disorders further encompasses treatment of any of the aforementioned conditions, symptoms and/or syndromes when caused by or associated with cystitis, including interstitial cystitis, prostatitis, BPH, neurological disorders, decreased urinary compliance (i.e., decreased bladder storage capacity).

In certain preferred aspects of the invention, a combination of at least one A2d ligand and at least one NSAID is used to treat the involuntary passage of urine, i.e., urinary incontinence, e.g., urge incontinence, stress incontinence, mixed incontinence or overflow incontinence. In further preferred aspects of the invention, a combination of at least one A2d ligand and at least one NSAID, is used to treat the involuntary passage of urine, i.e., urinary incontinence, e.g., urge incontinence, stress incontinence, mixed incontinence or overflow incontinence, that is caused by and/or associated with OAB or BPH.

A2d ligands for use in the invention are gabapentin (US 4024175), pregabalin (EP 0641330), (1R,5R,6S)-6-aminomethyl-6-carboxymethyl-bicyclo[3.2.0]heptane (WO 02/085839), 3-(1-aminomethyl-cyclohexylmethyl)-4H-1,2,4-oxadiazol-5-one (WO 99/31075), 5-(1-aminomethyl-cyclohexylmethyl)-1H-tetrazole (WO 99/31075), (3S,4S)-(1-aminomethyl-1-carboxymethyl-3,4-dimethyl-cyclopentane (WO 99/21824), (1α,3α,5α)-(3-aminomethyl-3-carboxymethyl-bicyclo[3.2.0]heptane (WO 01/28978), (3S,5R)-3-aminomethyl-5-methyl-octanoic acid (WO 00/76958), (3S,5R)-3-amino-5-methyl-heptanoic acid (WO 2003/082807), (3S,5R)-3-amino-5-methyl-octanoic acid (WO 2003/082807), (3S,5R)-3-amino-5-methyl-nonanoic acid (WO 2003/082807), (2S,4S)-4-(3-chlorophenoxy)-proline or (2S,4S)-4-(3-fluorobenzyl)-proline, or pharmaceutically acceptable salts thereof. Particularly preferred A2d ligands are selected from gabapentin, pregabalin and ((1α,3α,5α)-(3-aminomethyl-3-carboxymethyl-bicyclo[3.2.0]heptane, or pharmaceutically acceptable salts thereof.

NSAIDs useful for combination treatment with A2d ligands include:
(i) ibuprofen, naproxen, benoxaprofen, flurbiprofen, fenoprofen, fenbufen, ketoprofen, indoprofen, pirprofen, carprofen, oxaprozin, prapoprofen, miroprofen, tioxaprofen, suprofen, alminoprofen, tiaprofenic acid, fluprofen, bucloxic acid, indomethacin, sulindac, tolmetin, zomepirac, diclofenac, fenclofenac, alclofenac, ibufenac, isoxepac, furofenac, tiopinac, zidometacin, acetyl salicylic acid, piroxicam, tenoxicam, nabumetone, ketorolac, azapropazone, mefenamic acid, tolfenamic acid, diflunisal, podophyllotoxin derivatives, droxicam, floctafenine, oxyphenbutazone, phenylbutazone, proglumetacin, acemetacin, fentiazac, clidanac, oxipinac, meclofenamic acid, flufenamic acid, niflumic acid, flufenisal, sudoxicam, etodolac, salicylic acid, choline magnesium trisalicylate, salicylate, benorylate, clopinac, feprazone, isoxicam and 2-fluoro-a-methyl[1,1'-biphenyl]-4-acetic acid, 4-(nitrooxy)butyl ester (See Wenk, et al., Europ. J. Pharmacol. 453:319-324 (2002));
(ii) meloxicam, (CAS registry number 71125-38-7; described in US 4233299), or a pharmaceutically acceptable salt or prodrug thereof;
(iii) Substituted benzopyran derivatives that are described in US 6271253. Also benzopyran derivatives described in US 6034256 and US 6077850 along with WO 98/47890 and WO 00/23433;
(iv) Chromene COX2 selective inhibitors described in US 6077850 and US 6034256;
(v) The compounds described in WO 95/30656, WO 95/30652, WO 96/38418 and WO 96/38442, and the compounds described in EP 0799823, along with the pharmaceutically acceptable derivatives thereof;
(vi) celecoxib (US 5466823), valdecoxib (US 5633272), deracoxib (US 5521207), rofecoxib (US 5474995), etoricoxib (WO 98/03484), JTE-522 (JP 9052882), or a pharmaceutically acceptable salt or prodrug thereof;
(vii) Parecoxib (described in US 5932598), which is a therapeutically effective prodrug of the tricyclic Cox-2 selective inhibitor valdecoxib (described in US 5633272), in particular sodium parecoxib;
(viii) ABT-963 (described in WO 00/24719)
(ix) Nimesulide (described in US 3840597), flosulide (discussed in J. Carter, Exp.Orin.Ther.Patents, 8(1), 21-29 (1997)), NS-398 (disclosed in US 4885367), SD 8381 (described in US 6034256), BMS-347070 (described in US 6180651), S-2474 (described in EP 0595546) and MK-966 (described in US 5968974);
(x) The compounds and pharmaceutically acceptable derivatives described in US 6395724, US 6077868, US 5994381, US 6362209, US 6080876, US 6133292, US 6369275, US 6127545, US 6130334, US 6204387, US 6071936, US 6001843, US 6040450, WO 96/03392, WO 96/24585, US 6340694, US 6376519, US 6153787, US 6046217, US 6329421, US 6239137, US 6136831, US 6297282, US 6239173, US 6303628, US 6310079, US 6300363, US 6077869, US 6140515, US 5994379, US 6028202, US 6040320, US 6083969, US 6306890, US 6307047, US 6004948, US 6169188, US 6020343, US 5981576, US 6222048, US 6057319, US 6046236, US 6002014, US 5945539, US 6359182, WO 97/13755, WO 96/25928, WO 96/374679, WO 95/15316, WO 95/15315, WO 96/03385, WO 95/00501, WO 94/15932, WO 95/00501, WO 94/27980, WO 96/25405, WO 96/03388, WO 96/03387, US 5344991, WO 95/00501, WO 96/16934, WO 96/03392, WO 96/09304, WO 98/47890 and WO 00/24719.

In certain embodiments, NSAIDs for use in the present invention may be non-selective cyclooxygenase (COX) inhibitors, i.e., compounds that inhibit both COX-1 and COX-2 proteins, or may be selective COX-2 inhibitors. Classes of non-selective COX inhibitors include salicylic acid derivatives (e.g., aspirin, sodium salicylate, choline magnesium trisalicylate, salsalate, diflunisal, sulfasalazine and olsalazine), para-aminophenol derivatives (e.g., acetaminophen), indole and indene acetic acids (e.g., indomethacin and sulindac), heteroaryl acetic acids (e.g., tolmetin, diclofenac and ketorolac), arylpropionic acids (e.g., ibuprofen, naproxen, flurbiprofen, ketoprofen, fenoprofen, and oxaprozin), anthranilic acids (fenamates) (e.g., mefenamic acid and meclofenamic acid), enolic acids (e.g., the oxicams, piroxicam and meloxicam) and alkanones (e.g., nabumetone). Selective COX-2 inhibitors include diaryl-substituted furanones (e.g., rofecoxib), diaryl-substituted pyrazoles (e.g., celecoxib), indole acetic acids (e.g., etodolac) and sulfonanilides (e.g., nimesulide). Further examples of selective COX-2 inhibitors are disclosed in US 6440963 and WO 2004/054560.

The combination therapy enabled by the various embodiments of the invention as set out above is not limited to two active ingredients, the A2d ligand and the NSAID. Further active ingredients may be incorporated. Examples of such further active ingredients are antimuscarinic agents, α₁-adrenergic antagonists, and serotonin and/or noradrenaline reuptake inhibitors.

Examples of suitable antimuscarinic agents are oxybutynin, tolterodine, darifenacin, solifenacin, trospium, fesoterodine and temiverine.

Examples of suitable α₁-adrenergic antagonist are prazosin, doxazosin, terazosin, alfuzosin, silodosin, and tamsulosin. Additional α₁-adrenergic antagonists suitable for administration in combination with A2d ligands and NSAIDs are described in US 5990114, US 6306861, US 6365591, US 6387909 and US 6403594.

Examples of suitable serotonin and/or noradrenaline reuptake inhibitors are tandamine, amoxapine, pirandamine, ciclazindole, fluparoxane, lortalamine, talsupram, talopram, prindamine, nomifensin, viloxazine, tomoxetine, duloxetine, venlafaxine, des-venlafaxine, milnacipran, tesofensine, reboxetine, sibutramine, sibutramine hydrochloride, (R-) or (S-)-didesmethylsibutramine, (R-) or (S-)-desmethylsibutramine, desipramine, maprotiline, nomifensin, citalopram, fluoxetine, fluvoxamine, paroxetine, sertraline and trazodone. Additional serotonin and/or noradrenaline reuptake inhibitors suitable for administration in combination with A2d ligands and NSAIDs are disclosed in EP 1220831, EP 1154984, US 4018830, US 5190965, US 5430063, US 4161529, WO 97/17325 and U.S. provisional application 60/121,313. Preferred serotonin and/or noradrenaline reuptake inhibitors suitable for administration in combination with A2d ligands and NSAIDs are duloxetine, milnacipran, amoxapine, venlafaxine, des-venlafaxine, sibutramine, tesofensine and des-methylsibutramine.

In certain embodiments, a serotonin and/or noradrenaline reuptake inhibitor suitable for administration in combination with A2d ligands and NSAIDs is a selective serotonin reuptake inhibitor (i.e., an SSRI). In certain embodiments, a serotonin and/or noradrenaline reuptake inhibitors suitable for administration in combination with A2d ligands and NSAIDs is a selective noradrenaline reuptake inhibitor (i.e., an NARI).

A chemical antagonist is a substance wherein the antagonist binds the ligand in solution so the effect of the ligand is lost. A pharmacokinetic antagonist is one which effectively reduces the concentration of the active ligand at its site of action, for example, by increasing the rate of metabolic degradation of the active ligand. Antagonism by receptor-block involves two important mechanisms: reversible competitive antagonism and irreversible, or non-equilibrium competitive antagonism. Reversible competitive antagonism occurs when the rate of dissociation of the antagonist molecules is sufficiently high such that, on addition of the ligand, displacement of chemical antagonist molecules from the receptors effectively occurs. Of course the ligand cannot displace a bound antagonist molecule, or vice versa. Irreversible or non-equilibrium competitive antagonism occurs when the antagonist dissociates very slowly, or not at all, from the receptor with the result that no change in the antagonist occupancy takes place when the ligand is applied. Thus, the antagonism is irreversible. Non-competitive antagonism describes the situation where the antagonist blocks at some point in the signal transduction pathway leading to the production of a response by the ligand.

Physiological antagonism is a term used loosely to describe the interaction of two substances whose opposing actions in the body tend to cancel each other out. An antagonist can also be a substance which diminishes or abolishes expression of functional receptor. An inverse agonist is a substance which preferentially binds to the inactive state of the receptor (in contrast to the agonists that bind preferentially to the active state of the receptor), and therefore avoids the stimulation of the receptor by the agonist. In general, the in vivo activity of inverse agonists is similar to that of antagonists and for the sake of clarity inverse agonists will be defined as antagonists in the present application.

Without limiting the present disclosure, biological activity and/or potency of compounds useful in the present invention may be measured by determining the activity, e.g., binding of compounds in vivo or in vitro, including cell extracts or fractions of extracts. Potency may also be determined using native or recombinant receptors, that are expressed constitutively or that have been induced, and that have expressed in native or non-native species and/or cell types. The biological activity of compounds suitable for use in the invention as A2d ligands may be measured in a radioligand binding assay, e.g., using [³H]gabapentin and the α₂δ subunit derived from porcine brain tissues (Gee et al., J. Biol. Chem., 1996;271:5879-5776; US 6441156).

A2d ligands preferably bind with an affinity between about 1000 and 0.1 nM (measured as, e.g., IC₅₀, EC₅₀, Kᵢ, or K_{d}). The biological activity of NSAIDs may be measured, e.g., by measuring inhibition of COX-1 and COX-2 isozymes in CHO cells stably transfected with human COX-1 or COX-2 (Riendeau et al., Br. J. Pharmacol. 121:105, 1997; Elrich et al., Clin. Pharm. Ther. 65: 336, 1999) or inhibition of COX-1 and COX-2 isozymes in Sf9 cells (see US 6440963).

Once a compound is identified as an A2d ligand or an NSAID, its pharmacological activity can be confirmed using one or more animal model systems for neuromuscular dysfunction of the lower urinary tract, either alone or in combination with other agents.

An animal model for measuring pharmacological activity on the lower urinary tract is volume-induced rhythmic bladder voiding contractions in anesthetized rats. In this method, the urinary bladder is catheterized through the external urethra with a polyethylene tubing filled with physiological saline. The external urethra is then ligated and connected to a pressure recording device. The bladder is then filled with saline until reflex voiding contractions occur, after which the frequency of the voiding contractions is measured for 15 min. Test compounds are then administered intravenously and their effect evaluated for the following 60 min.. This model has been validated by the use of different reference standards (Guarneri et al., Pharmacol. Res. 27:173-187, 1993).

Other animal models useful to assess activity on the lower urinary tract are based on cystometric recording of bladder activity in conscious rats instrumented in order to measure bladder pressure during constant infusion of the bladder with saline or very diluted acetic acid. Velasco C. et al., J. Urol. 166: 1962-1968, 2001. These methods are widely used and accepted by researchers skilled in this field and foresee a period of infusion of about five hours after administration of test compounds with continuous monitoring of bladder performance and assessment of intervals between micturitions and peak micturition pressure.

Also within the scope of the invention are so-called "prodrugs" of compounds suitable for use in the present invention. Thus certain derivatives of compounds suitable for use in the present invention which may have little or no pharmacological activity themselves can, when administered into or onto the body, be converted into compounds having the desired activity, for example, by hydrolytic cleavage. Such derivatives are referred to as "prodrugs." Further information on the use of prodrugs may be found in Pro-drugs as Novel Delivery Systems, Vol. 14, ACS Symposium Series (T Higuchi and W Stella) and 'Bioreversible Carriers in Drug Design', Pergamon Press, 1987 (ed. E B Roche, American Pharmaceutical Association).

Prodrugs in accordance with the invention can, for example, be produced by replacing appropriate functionalities present in the compounds suitable for use in the present invention with certain moieties known to those skilled in the art as "pro-moieties," as described, for example, in "Design of Prodrugs" by H Bundgaard (Elsevier, 1985).

Some examples of prodrugs in accordance with the invention include:
(i) where the compound contains a carboxylic acid functionality (-COOH), an ester thereof, for example, replacement of the hydrogen with (C₁₋₈)alkyl;
(ii) where the compound contains an alcohol functionality (-OH), an ether thereof, for example, replacement of the hydrogen with (C₁₋₆)alkanoyloxymethyl; and
(iii) where the compound contains a primary or secondary amino functionality (-NH₂ or -NHR where R = alkyl), an amide thereof, for example, replacement of one or both hydrogens with (C₁₋₁₀)alkanoyl.

Further examples of replacement groups in accordance with the foregoing examples and examples of other prodrug types may be found in the aforementioned references.

Finally, certain compounds suitable for use in the present invention may themselves act as prodrugs of other compounds suitable for use in the present invention.

In another embodiment, the present invention provides pharmaceutical compositions comprising compounds having the same type of activity, enantiomers, diastereomers, N-oxides, crystalline forms, hydrates, solvates or pharmaceutically acceptable salts of such compounds, in admixture with pharmaceutically acceptable diluents or carriers such as those disclosed.

A metabolite of a compound disclosed herein is a derivative of a compound which is formed when the compound is metabolized. The term "active metabolite" refers to a biologically active derivative of a compound which is formed when the compound is metabolized.

The term "metabolized" refers to the sum of the processes by which a particular substance is changed in the living body. In brief, all compounds present in the body are manipulated by enzymes within the body in order to derive energy and/or to remove them from the body. Specific enzymes produce specific structural alterations to the compound. For example, cytochrome P450 catalyses a variety of oxidative and reductive reactions while uridine diphosphate glucuronyltransferases catalyze the transfer of an activated glucuronic-acid molecule to aromatic alcohols, aliphatic alcohols, carboxylic acids, amines and free sulphydryl groups. Further information on metabolism may be obtained from The Pharmacological Basis of Therapeutics, 9th Edition, McGraw-Hill (1996), pages 11-17.

Metabolites of the compounds disclosed herein can be identified either by administration of compounds to a host and analysis of tissue samples from the host, or by incubation of compounds with hepatic cells in vitro and analysis of the resulting compounds. Both methods are well known in the art.

### Pharmaceutical Compositions

The A2d ligand and/or NSAID may be presented as a pharmaceutical composition comprising the aforesaid A2d ligand and/or NSAID, or an enantiomer, diastereomer, N-oxide, crystalline form, hydrate, solvate, active metabolite or pharmaceutically acceptable salt of such a compound.

A pharmaceutical composition may also include optional additives, such as a pharmaceutically acceptable carrier or diluent, a flavoring, a sweetener, a preservative, a dye, a binder, a suspending agent, a dispersing agent, a colorant, a disintegrator, an excipient, a diluent, a lubricant, an absorption enhancer, a bactericide and the like, a stabilizer, a plasticizer, an edible oil, or any combination of two or more of said additives.

Suitable pharmaceutically acceptable carriers or diluents include ethanol, water, glycerol, aloe vera gel, allantoin, glycerine, vitamin-A and E oils, mineral oil, phosphate buffered saline, PPG2 myristyl propionate, magnesium carbonate, potassium phosphate, vegetable oil, animal oil and solketal.

Suitable binders include starch, gelatin, natural sugars such as glucose, sucrose and lactose, corn sweeteners, natural and synthetic gums such as acacia, tragacanth, vegetable gum, sodium alginate, carboxymethylcellulose, polyethylene glycol and waxes.

Suitable disintegrators include starch such as corn starch, methyl cellulose, agar, bentonite and xanthan gum.

Suitable lubricants include sodium oleate, sodium stearate, magnesium stearate, sodium benzoate, sodium acetate and sodium chloride.

Suitable suspending agents include bentonite.

Suitable dispersing and suspending agents include synthetic and natural gums such as vegetable gum, tragacanth, acacia, alginate, dextran, sodium carboxymethylcellulose, methylcellulose, polyvinylpyrrolidone and gelatin.

Suitable edible oils include cottonseed oil, sesame oil, coconut oil and peanut oil.

Examples of additional additives include sorbitol, talc, stearic acid and dicalcium phosphate.

In certain embodiments, pharmaceutical compositions comprise an A2d ligand, an NSAID and a pharmaceutically acceptable excipient. More preferably, such compositions comprise a first amount of said A2d ligand and a second amount of said NSAID that provide a synergistic effect in treatment of urinary incontinence when administered to an individual in need of treatment. More preferably, such compositions comprise a first amount of NSAID that is below an effective amount of said NSAID for monotherapy treatment of urinary incontinence and/or a first amount of A2d ligand that is below an effective amount of A2d ligand for monotherapy treatment of said urinary incontinence. Most preferably, such compositions comprise a first amount of A2d ligand that is below an effective amount of A2d ligand for monotherapy treatment of urinary incontinence and a second amount of NSAID that is below an effective amount of NSAID for monotherapy treatment of urinary incontinence.

As used herein, a "synergistic effect" refers to a superadditive effect obtained when agents are administered in combination, compared to when such agents are administered individually.

### Unit Dosage Forms

The pharmaceutical composition may be formulated as unit dosage forms, such as tablets, pills, capsules, boluses, powders, granules, sterile parenteral solutions, sterile parenteral suspensions, sterile parenteral emulsions, elixirs, tinctures, metered aerosol or liquid sprays, drops, ampoules, autoinjector devices or suppositories. The unit dosage forms may be used for oral, parenteral, intranasal, sublingual or rectal administration, or for administration by inhalation or insufflation, transdermal patches, and a lyophilized composition. In general, any delivery of active ingredients that results in systemic availability of such ingredients can be used. Preferably the unit dosage form is an oral dosage form, most preferably a solid oral dosage; therefore the preferred dosage forms are tablets, pills and capsules. However, parenteral preparations are preferred too.

Solid unit dosage forms may be prepared by mixing the active agents of the present invention with a pharmaceutically acceptable carrier and any other desired additives as described above. The mixture is typically mixed until a homogeneous mixture of the active agents of the present invention and the carrier and any other desired additives is obtained, i.e., the active agents are dispersed evenly throughout the composition. In this case, the composition can be formed as dry or moist granules.

Tablets or pills can be coated or otherwise prepared so as to form a unit dosage form that has delayed and/or sustained action, such as controlled release and delayed release unit dosage forms. For example, the tablet or pill can comprise an inner dosage and an outer dosage component, the latter being in the form of a layer or envelope over the former. The two components can be separated by an enteric layer which serves to resist disintegration in the stomach and permits the inner component to pass intact into the duodenum or to be delayed in release.

Biodegradable polymers for controlling the release of the active agents include polylactic acid, polyepsilon caprolactone, polyhydroxybutyric acid, polyorthoesters, polyacetals, polydihydropyrans, polycyanoacrylates and crosslinked or amphipathic block copolymers of hydrogels.

For liquid dosage forms, the active substances or their physiologically acceptable salts are dissolved, suspended or emulsified, optionally with the usually employed substances such as solubilizers, emulsifiers or other auxiliaries. Solvents for the active combinations and the corresponding physiologically acceptable salts can include water, physiological salt solutions or alcohols, e.g., ethanol, propanediol or glycerol. Additionally, sugar solutions such as glucose or mannitol solutions may be used. A mixture of the various solvents mentioned may be used in the present invention too.

A transdermal dosage form is contemplated by the present invention too. Transdermal forms may be a diffusion transdermal system (transdermal patch) using either a fluid reservoir or a drug-in-adhesive matrix system. Other transdermal dosage forms include topical gels, lotions, ointments, transmucosal systems and devices, and iontophoretic (electrical diffusion) delivery systems. Transdermal dosage forms may be used for delayed release and sustained release of the active agents of the present invention.

The pharmaceutical compositions and unit dosage forms of the present invention for parenteral administration, and in particular by injection, typically include a pharmaceutically acceptable carrier, as described above. A preferred liquid carrier is vegetable oil. Injection may be, for example, intravenous, epidural, intrathecal, intramuscular, intraluminal, intratracheal or subcutaneous.

The active agents can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active agents of the present invention may also be coupled with soluble polymers such as targetable drug carriers. Such polymers include polyvinylpyrrolidone, pyran copolymers, polyhydroxypropylmethacrylamidophenol, polyhydroxyethylaspartamidophenol, and polyethylenoxypolylysine substituted with palmitoyl residues.

### Administration

The pharmaceutical composition or unit dosage forms may be administered by a variety of routes, such as the oral and enteral, intravenous, intramuscular subcutaneous, transdermal, transmucosal (including rectal and buccal) and by inhalation routes. Preferably, the oral or transdermal route is used (i.e., with solid or liquid formulations or skin patches, respectively).

### Dosage Amounts

As used herein, the term "effective amount" refers to an amount that results in measurable amelioration of at least one symptom or parameter of a specific disorder.

The pharmaceutical composition or unit dosage form may be administered according to a dosage and administration regimen defined by routine testing in light of the guidelines given above in order to obtain optimal activity while minimizing toxicity or side effects for a particular patient. However, such fine tuning of the therapeutic regimen is routine in the light of the guidelines given herein.

The dosage of the active agents of the present invention may vary according to a variety of factors such as underlying disease conditions, the individual's condition, weight, sex and age, and the mode of administration. An effective amount for treating a disorder can easily be determined by empirical methods known to those of ordinary skill in the art, for example by establishing a matrix of dosages and frequencies of administration and comparing a group of experimental units or subjects at each point in the matrix. The exact amount to be administered to a patient will vary depending on the state and severity of the disorder and the physical condition of the patient. A measurable amelioration of any symptom or parameter can be determined by a person skilled in the art or reported by the patient to the physician. It will be understood that any clinically or statistically significant attenuation or amelioration of any symptom or parameter of urinary tract disorders is within the scope of the invention. Clinically significant attenuation or amelioration means perceptible to the patient and/or to the physician.

For example, a single patient may suffer from several symptoms of dysuria simultaneously, such as, for example, urgency and excessive frequency of urination or both, and these may be reduced using the methods of the present invention. In the case of incontinence, any reduction in the frequency or volume of unwanted passage of urine is considered a beneficial effect of the present method of treatment.

In the disclosed compositions, the A2d ligand and the NSAID together provide a therapeutically effective amount of an active combination of agents. Each agent present in a therapeutically effective amount of an active combination of agents may itself be present in a therapeutically effective amount or in a suboptimal amount (i.e., in an amount that is below a therapeutically effective amount, e.g., when used in monotherapy). A therapeutically effective amount of an active combination of A2d ligand and NSAID, for example, may include each of A2d ligand and NSAID in therapeutically effective amounts, or one or both of them may be in a suboptimal amount. The optimal therapeutically effective amount of an active combination of agents should be determined experimentally, taking into consideration the exact mode of administration, form in which the drug is administered, the indication toward which the administration is directed, the subject involved (e.g., body weight, health, age, sex, responsiveness to a particular therapeutic agent, etc.), and the preference and experience of the physician or veterinarian in charge. As described herein, since there is a synergistic effect of combination treatment with A2d and NSAID, one or both will be effective in the combination at an amount lower than its corresponding effective amount as monotherapy.

Drug combinations of, e.g., A2d ligands and NSAIDs, are not only highly effective at relatively low doses but also possesses low toxicity and produces few side effects. The amount of an active agent to be administered can typically range between about 0.01 and about 50 mg/kg/day, preferably between about 0.1 and about 25 mg/kg/day and more preferably between 0.2 and about 12 mg/kg/day. Total amount of active agent administered daily can typically range between about 0.75 and about 3750 mg, preferably between about 7.5 and about 1875 mg, and more preferably between about 15 and about 900 mg. It will be understood that the pharmaceutical formulations of the present invention need not necessarily contain the entire amount of the agent that is effective in treating the disorder, as such effective amounts can be reached by administration of a plurality of doses of such pharmaceutical formulations.

In a preferred embodiment of the present invention, the compounds are formulated in capsules or tablets, preferably containing 50 to 200 mg of the compounds of the invention, and are preferably administered to a patient at a total daily dose of 50 to 400 mg, preferably 150 to 250 mg and most preferably about 200 mg, for relief of lower urinary tract disorders under treatment with an A2d ligand and an NSAID.

A pharmaceutical composition for parenteral administration contains from about 0.01% to about 100% by weight of the active agents of the present invention, based upon 100% weight of total pharmaceutical composition.

Generally, transdermal dosage forms contain from about 0.01% to about 100% by weight of the active agents versus 100% total weight of the dosage form.

### Combination therapy

As used herein, the term "combination" used in conjunction with the terms therapy, treatment, or administration refers to therapy, treatment or administration of, or with a first amount of an A2d ligand and a second amount of an NSAID, wherein the first and second amounts together comprise a therapeutically effective amount to treat at least one symptom of a lower urinary tract disorder in a subject in need of treatment. The term combination encompasses administration of first and second amounts of compounds in an essentially concomitant manner, such as in a single pharmaceutical composition, for example, capsule or tablet having a fixed ratio of first and second amounts, or in multiple, separate capsules or tablets for each. The term combination also encompasses administration of first and second amounts of compound in a sequential manner, in either order. When combination treatment involves the sequential administration of the first amount of, e.g., an A2d ligand and the second amount of an NSAID the compounds are administered sufficiently close in time to have the desired therapeutic effect. For example, the period of time between each administration which can result in the desired therapeutic effect, can range from minutes to hours and can be determined taking into account the properties of each compound such as potency, solubility, bioavailability, plasma half-life and kinetic profile. For example, an A2d ligand and NSAID can be administered in any order within about 16 hours of each other, within about 8 hours of each other, within about 4 hours of each other, within about 1 hour of each other, within about 30 minutes of each other or within about 10 minutes of each other.

For combination therapy with A2d ligand and NSAID, the ratio of daily dosage of A2d:NSAID is preferably in the range of about 1:1 to about 200:1 w/w (i.e., by weight ratio). Preferred ratios of A2d:NSAID administered in combination treatment are about 100:1, 50:1, 40:1, 30:1, 20:1, 10:1, 5:1, 2:1 and 1:1. A further preferred ratio of A2d:NSAID administered in combination treatment is 30:1, and a further preferred ratio of A2d:NSAID administered in combination treatment is 10:1.

In certain embodiments, the daily dosages of A2d ligand and NSAID administered in combination to treat lower urinary tract disorders, e.g., urinary incontinence are 10-20 mg of A2d ligand and 5-10 mg of NSAID; 20-50 mg of A2d ligand and 5-10 mg of NSAID; 50-100 mg of A2d ligand and 5-10 mg of NSAID; 100-300 mg of A2d ligand and 5-10 mg of NSAID; 300-600 mg of A2d ligand and 5-10 mg of NSAID; 600-900 mg of A2d ligand and 5-10 mg of NSAID; 900-1200 mg of A2d ligand and 5-10 mg of NSAID; 1200-1500 mg of A2d ligand and 5-10 mg of NSAID; 1500-1800 mg of A2d ligand and 5-10 mg of NSAID; and 1800-2100 mg of A2d ligand and 5-10 mg of NSAID.

In certain embodiments, the daily dosages of A2d ligand and NSAID administered in combination to treat lower urinary tract disorders, e.g., urinary incontinence are 10-20 mg of A2d ligand and 10-20 mg of NSAID; 20-50 mg of A2d ligand and 20-50 mg of NSAID; 50-100 mg of A2d ligand and 25-50 mg of NSAID; 100-300 mg of A2d ligand and 25-50 mg of NSAID; 300-600 mg of A2d ligand and 25-50 mg of NSAID; 600-900 mg of A2d ligand and 25-50 mg of NSAID; 900-1200 mg of A2d ligand and 25-50 mg of NSAID; 1200-1500 mg of A2d ligand and 25-50 mg of NSAID; 1500-1800 mg of A2d ligand and 25-50 mg of NSAID; 1800-2100 mg of A2d ligand and 25-50 mg of NSAID; and 2100-2400 mg of A2d ligand and 25-50 mg of NSAID.

In certain embodiments, the daily dosages of A2d ligand and NSAID administered in combination to treat lower urinary tract disorders, e.g., urinary incontinence are 50-100 mg of A2d ligand and 50-100 mg of NSAID; 100-300 mg of A2d ligand and 50-100 mg of NSAID; 300-600 mg of A2d ligand and 50-100 mg of NSAID; 600-900 mg of A2d ligand and 50-100 mg of NSAID; 900-1200 mg of A2d ligand and 50-100 mg of NSAID; 1200-1500 mg of A2d ligand and 50-100 mg of NSAID; 1500-1800 mg of A2d ligand and 50-100 mg of NSAID; 1800-2100 mg of A2d ligand and 50-100 mg of NSAID; and 2100-2400 mg of A2d ligand and 50-100 mg of NSAID.

In certain embodiments, the daily dosages of A2d ligand and NSAID administered in combination to treat lower urinary tract disorders, e.g., urinary incontinence are 100-300 mg of A2d ligand and 100-300 mg of NSAID; 300-600 mg of A2d ligand and 100-500 mg of NSAID and 600-900 mg of A2d ligand and 100-500 mg of NSAID; 900-1200 mg of A2d ligand and 100-500 mg of NSAID; 1200-1500 mg of A2d ligand and 100-500 mg of NSAID; 1500-1800 mg of A2d ligand and 100-500 mg of NSAID; 1800-2100 mg of A2d ligand and 100-500 mg of NSAID; and 2100-2400 mg of A2d ligand and 100-500 mg of NSAID.

In certain embodiments, the daily dosages of A2d ligand and NSAID administered in combination to treat lower urinary tract disorders, e.g., urinary incontinence are 300-600 mg of A2d ligand and 300-600 mg of NSAID; 600-900 mg of A2d ligand and 500-1000 mg of NSAID; 900-1200 mg of A2d ligand and 500-1000 mg of NSAID; 1200-1500 mg of A2d ligand and 500-1000 mg of NSAID; 1500-1800 mg of A2d ligand and 500-1000 mg of NSAID; 1800-2100 mg of A2d ligand and 500-1000 mg of NSAID; and 2100-2400 mg of A2d ligand and 500-1000 mg of NSAID.

In preferred embodiments a combination of at least one A2d and at least one NSAID that is administered to treat lower urinary tract disorders, e.g., urinary incontinence, comprises gabapentin and an NSAID administered at one of the foregoing combinations of daily dosages. Further preferred is a combination a gabapentin and an NSAID selected from diclofenac, flurbiprofen, naproxen, nimesulide, celecoxib, sulindac, and diflunisal administered at one of the foregoing combinations of daily dosages. Further preferred is a combination of gabapentin and an NSAID selected from naproxen, flurbiprofen, nimesulide and diflunisal.

A pharmaceutical composition or unit dosage form may be administered in a single daily dose, or the total daily dosage may be administered in divided doses. In addition, simultaneous or sequential administration of another compound for the treatment of the disorder may be desirable. The order of administration will depend upon a variety of factors including age, weight, sex and medical condition of the patient; the severity and etiology of the disorders to be treated, the route of administration, the renal and hepatic function of the patient, the treatment history of the patient, and the responsiveness of the patient. Determination of the order of administration may be fine-tuned and such fine-tuning is routine in the light of the guidelines given herein.

### Packaged Kits

In another embodiment, a packaged kit is provided that contains one or more pharmaceutical formulation to be administered, i.e., one or more pharmaceutical formulation containing an A2d ligand and an NSAID to be administered in combination to treat lower urinary tract disorder, a container, preferably sealed, for housing the one or more formulation during storage and prior to use, and instructions for carrying out drug administration in a manner effective to treat a treat lower urinary tract disorder in a patient. The instructions will typically be written instructions on a package insert and/or on a label. Depending on the type of formulation and the intended mode of administration, the kit may also include a device for administering the formulation. The formulation may be any suitable formulation as described herein. For example, the formulation may be an oral dosage form containing a unit dosage of selected active agents. The kit may contain multiple formulations of different dosages of the same agent. The kit may also contain multiple formulations of different active agents. If more than one formulation comprising is present, for example in order to provide for repeat dosing, such formulations may be the same, or may be different in terms of the dosage, chemical composition and/or physical form.

Unless context dictates otherwise, the ratios of compounds set forth herein refer to relative amounts on a weight to weight basis (w/w).

### Brief Description of the Drawings

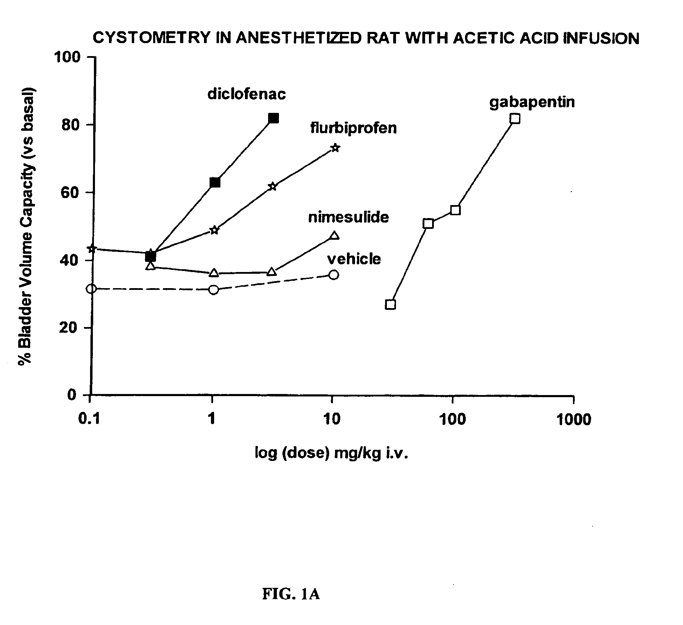

Fig. 1A shows change in bladder capacity following treatment with vehicle (circle), gabapentin (open square), diclofenac (solid square), flurbiprofen (star), and nimesulide (triangle). Results are expressed as % bladder volume capacity versus basal values (bladder infusion with saline).

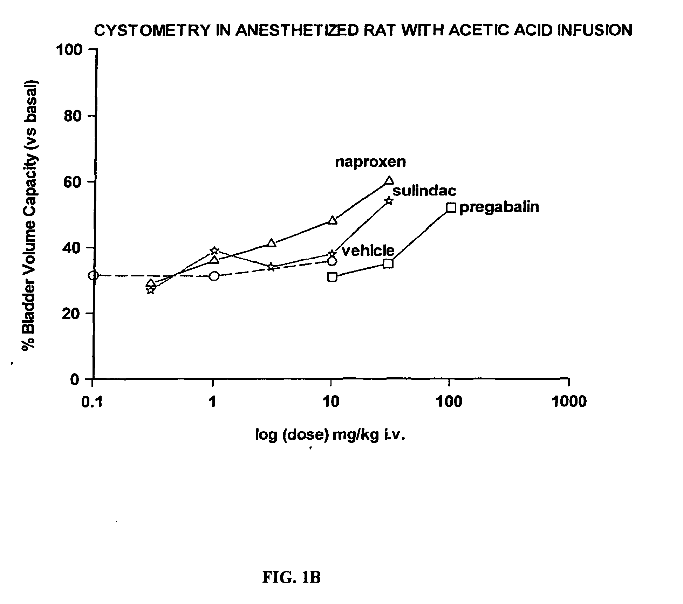

Fig. 1B shows change in bladder capacity following treatment with vehicle (circle), pregabalin (open square), naproxen (triangle) and sulindac (star). Results are expressed as % bladder volume capacity versus basal values (bladder infusion with saline).

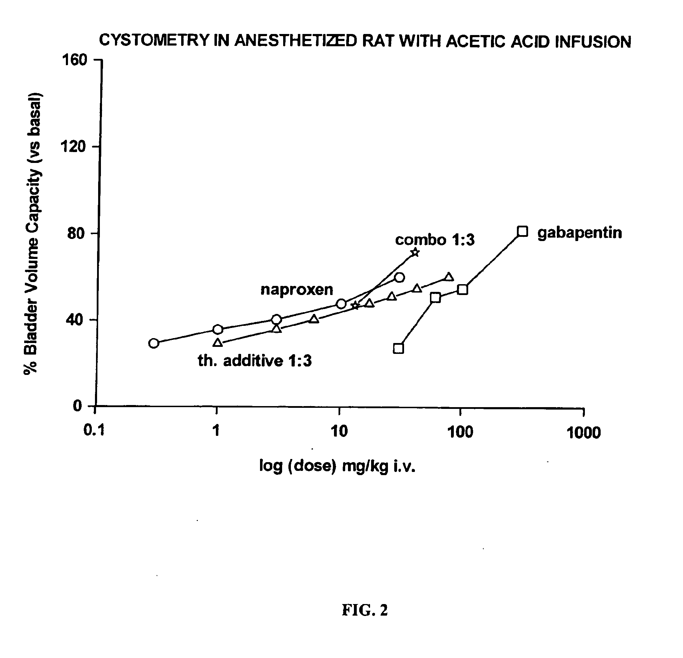

Fig. 2 shows change in bladder capacity following treatment with naproxen (circle), gabapentin (square) or naproxen:gabapentin combination (1:3 fixed dose ratio) (star) and theoretical additive curve calculated from single dose-response data (triangle). Results are expressed as % bladder volume capacity versus basal values (bladder infusion with saline).

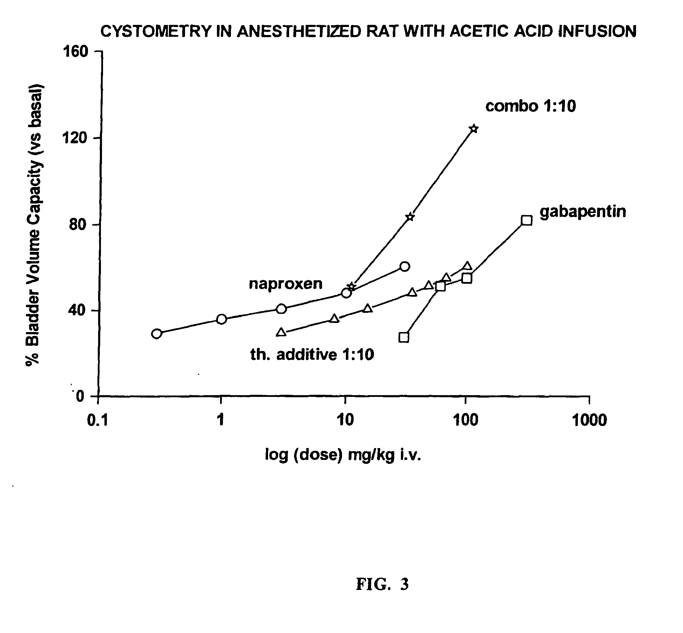

Fig. 3 shows change in bladder capacity following treatment with naproxen (circle), gabapentin (square) or naproxen:gabapentin combination (1:10 fixed dose ratio) (star) and theoretical additive curve calculated from single dose-response data (triangle). Results are expressed as % bladder volume capacity versus basal values (bladder infusion with saline).

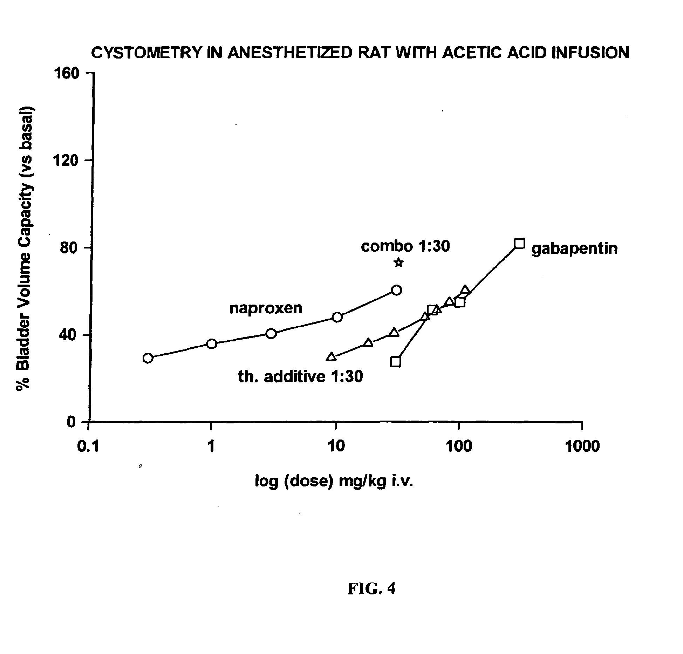

Fig. 4 shows change in bladder capacity following treatment with naproxen (circle), gabapentin (square) or naproxen:gabapentin combination (1:30 fixed dose ratio) (star) and theoretical additive curve calculated from single dose-response data (triangle). Results are expressed as % bladder volume capacity versus basal values (bladder infusion with saline).

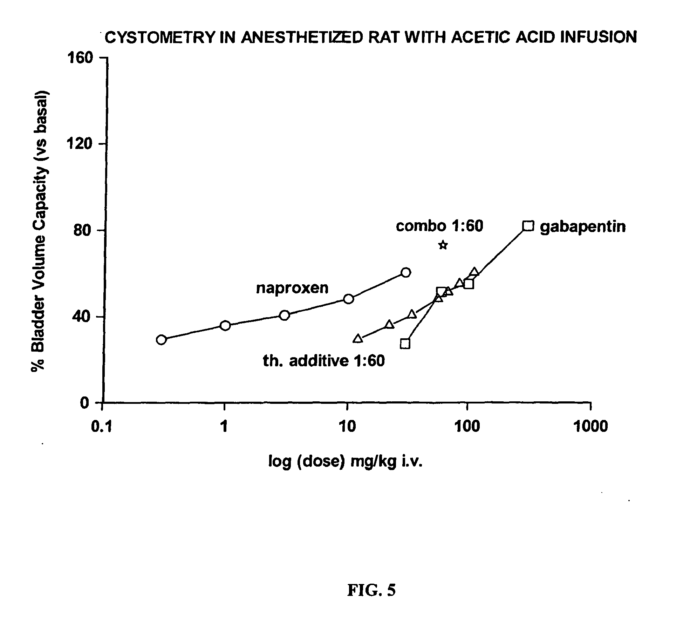

Fig. 5 shows change in bladder capacity following treatment with naproxen (circle), gabapentin (square) or naproxen:gabapentin combination (1:60 fixed dose ratio) (star) and theoretical additive curve calculated from single dose-response data (triangle). Results are expressed as % bladder volume capacity versus basal values (bladder infusion with saline).

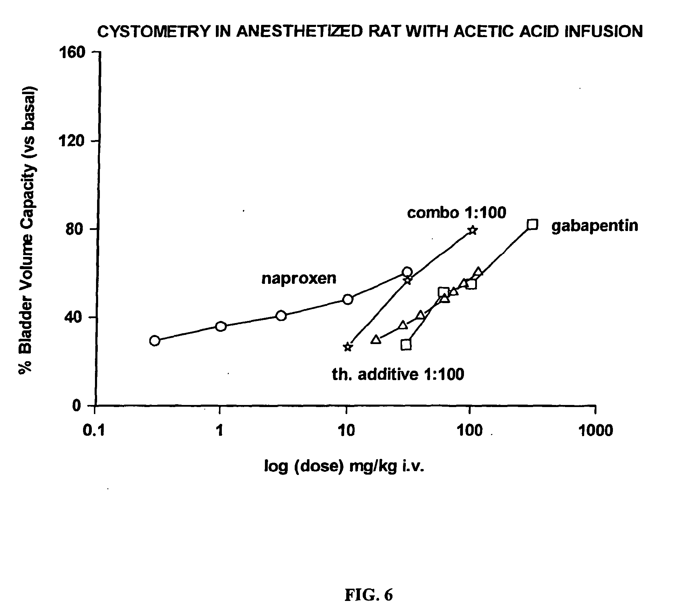

Fig. 6 shows change in bladder capacity following treatment with naproxen (circle), gabapentin (square) or naproxen:gabapentin combination (1:100 fixed dose ratio) (star) and theoretical additive curve calculated from single dose-response data (triangle). Results are expressed as % bladder volume capacity versus basal values (bladder infusion with saline).

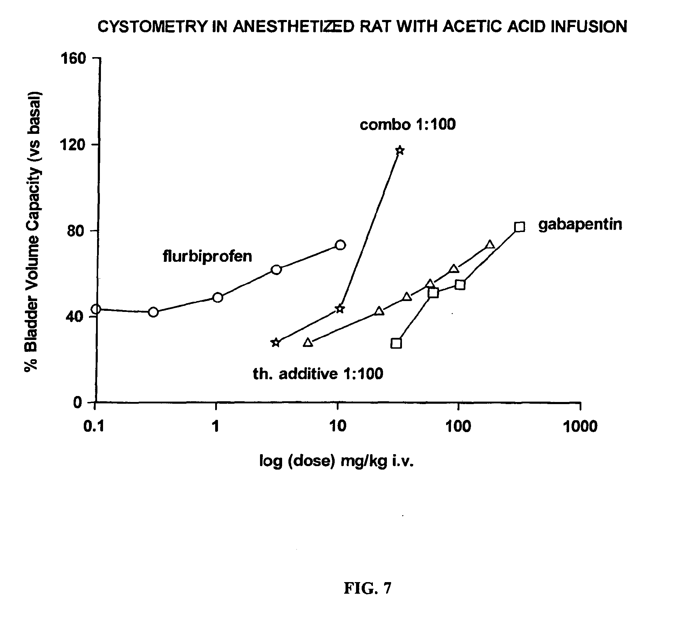

Fig. 7 shows change in bladder capacity following treatment with flurbiprofen (circle), gabapentin (square) or flurbiprofen:gabapentin combination (1:100 fixed dose ratio) (star) and theoretical additive curve calculated from single dose-response data (triangle). Results are expressed as % bladder volume capacity versus basal values (bladder infusion with saline).

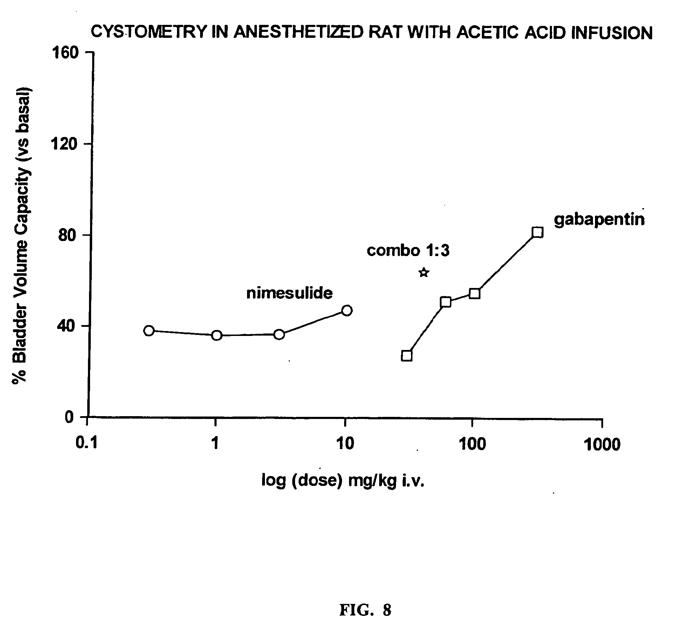

Fig. 8 shows change in bladder capacity following treatment with nimesulide (circle), gabapentin (square) or nimesulide:gabapentin combination (1:3 fixed dose ratio) (star). Results are expressed as % bladder volume capacity versus basal values (bladder infusion with saline).

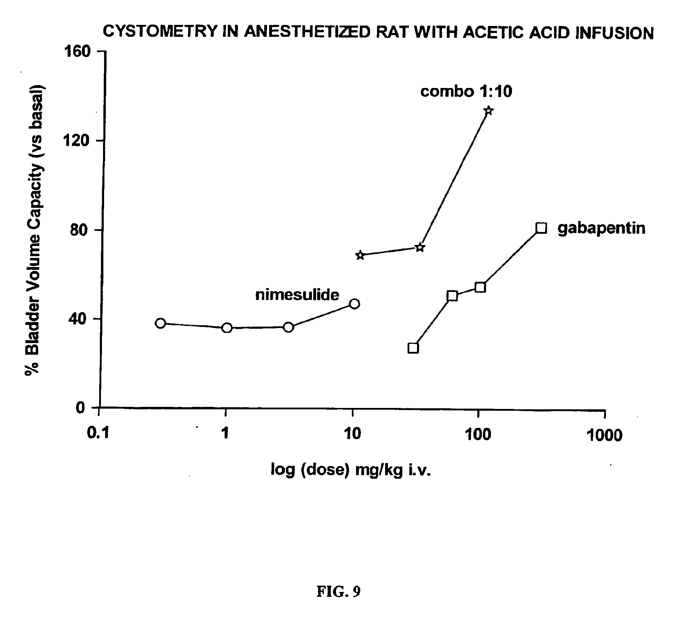

Fig. 9 shows change in bladder capacity following treatment with nimesulide (circle), gabapentin (square) or nimesulide:gabapentin combination (1:10 fixed dose ratio) (star). Results are expressed as % bladder volume capacity versus basal values (bladder infusion with saline).

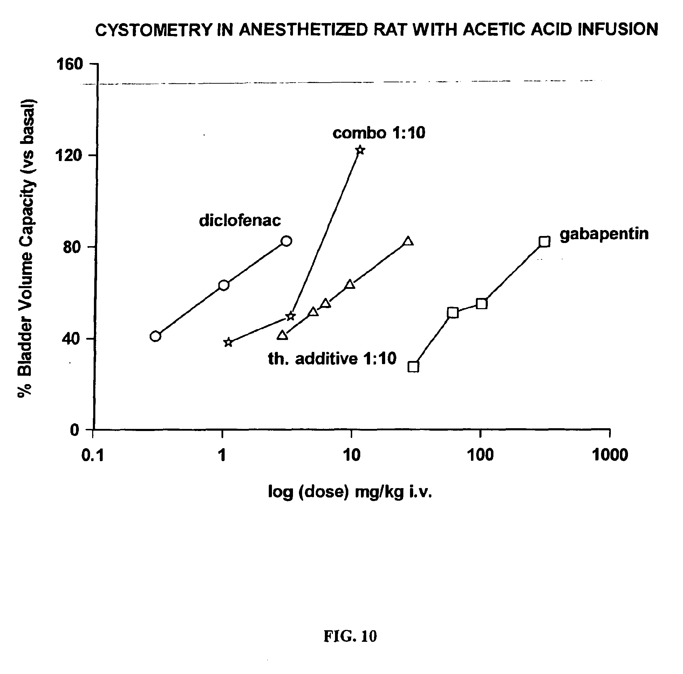

Fig. 10 shows change in bladder capacity following treatment with diclofenac (circle), gabapentin (square) or diclofenac:gabapentin combination (1:10 fixed dose ratio) (star) and theoretical additive curve calculated from single dose-response data (triangle). Results are expressed as % bladder volume capacity versus basal values (bladder infusion with saline).

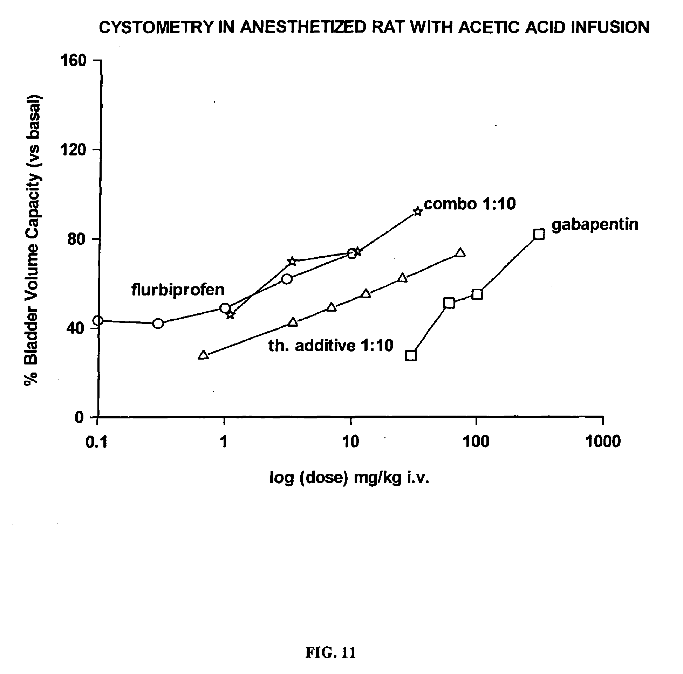

Fig. 11 shows change in bladder capacity following treatment with flurbiprofen (circle), gabapentin (square) or flurbiprofen:gabapentin combination (1:10 fixed dose ratio) (star) and theoretical additive curve calculated from single dose-response data (triangle). Results are expressed as % bladder volume capacity versus basal values (bladder infusion with saline).

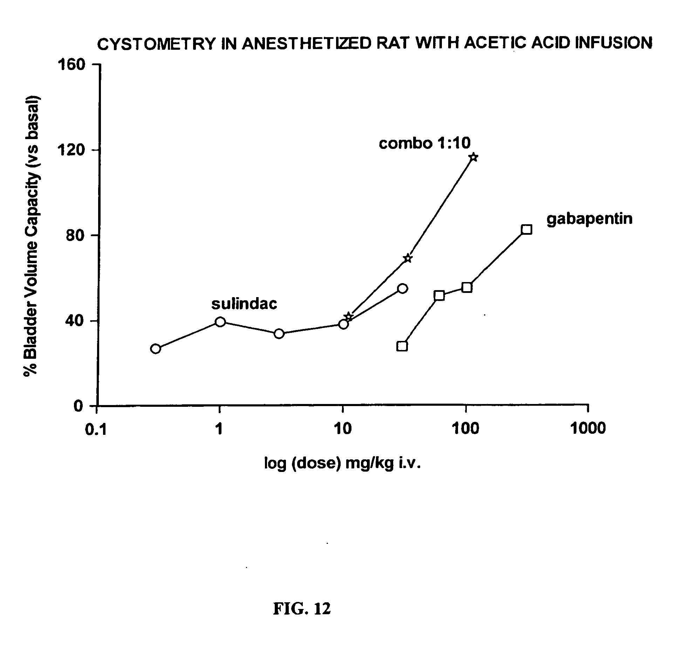

Fig. 12 shows change in bladder capacity following treatment with sulindac (circle), gabapentin (square) or sulindac:gabapentin combination (1:10 fixed dose ratio) (star). Results are expressed as % bladder volume capacity versus basal values (bladder infusion with saline).

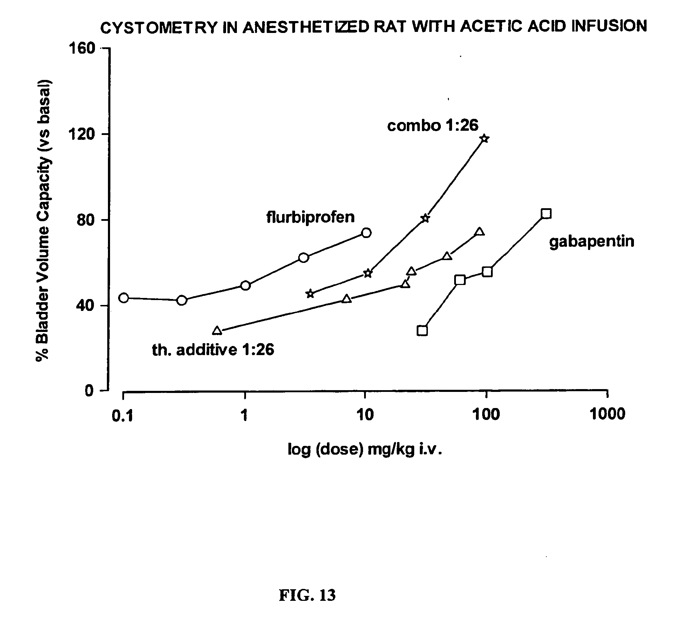

Fig. 13 shows change in bladder capacity following treatment with flurbiprofen (circle), gabapentin (square) or flurbiprofen:gabapentin combination (1:26 fixed dose ratio) (star) and theoretical additive curve calculated from single dose-response data (triangle). Results are expressed as % bladder volume capacity versus basal values (bladder infusion with saline).

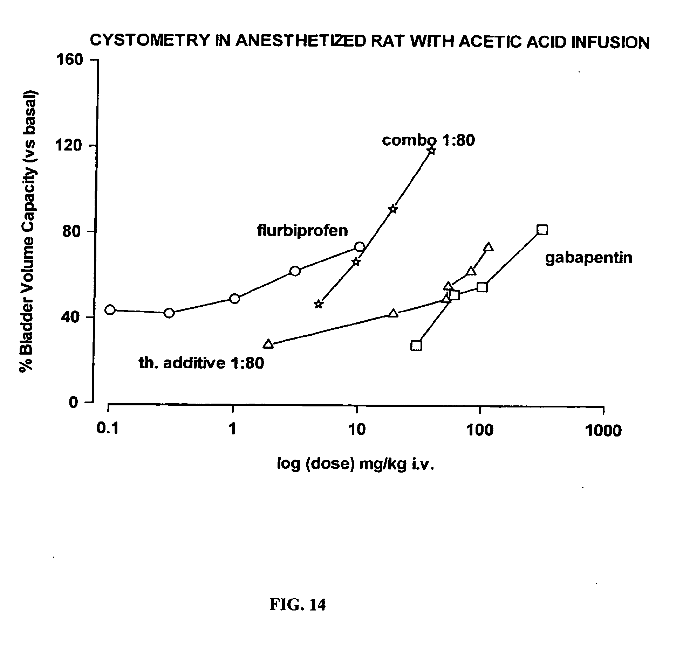

Fig. 14 shows change in bladder capacity following treatment with flurbiprofen (circle), gabapentin (square) or flurbiprofen:gabapentin combination (1:80 fixed dose ratio) (star) and theoretical additive curve calculated from single dose-response data (triangle). Results are expressed as % bladder volume capacity versus basal values (bladder infusion with saline).

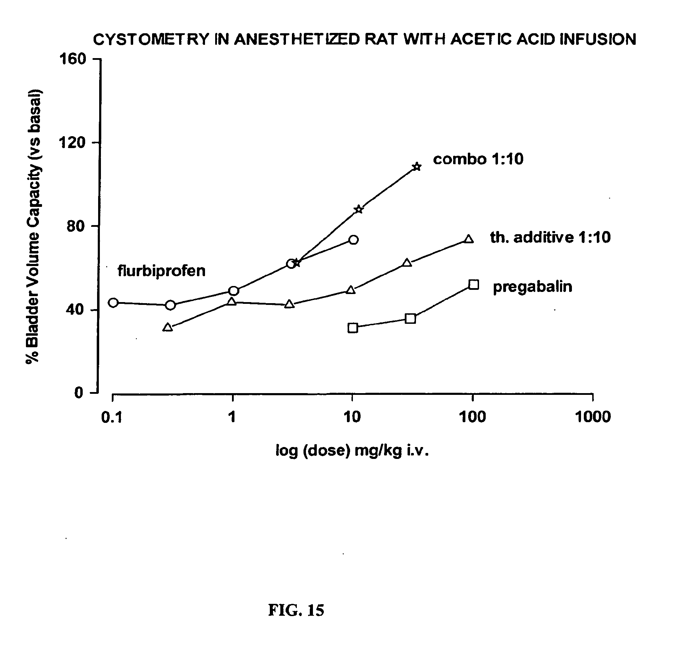

Fig. 15 shows change in bladder capacity following treatment with flurbiprofen (circle), pregabalin (square) or flurbiprofen:pregabalin combination (1:10 fixed dose ratio) (star) and theoretical additive curve calculated from single dose-response data (triangle). Results are expressed as % bladder volume capacity versus basal values (bladder infusion with saline).

### EXAMPLES

### Example 1. Cystometry in Anesthetized Rats With Acetic Acid Bladder Infusion

Animal Preparation. Female rats (225-250 g body weight) were anesthetized with urethane (1.25 g/kg s.c.) and a catheter (PE-50, 0.58 mm I.D. x 0.96 mm O.D.) was inserted into the jugular vein for intravenous drug administration. A midline lower abdominal incision was made and a second PE-50 catheter was inserted into the bladder dome for bladder filling and pressure recording, and the incision was sutured.

Groups of rats were treated with a NSAID alone (n = 6-8) and gabapentin or pregabalin alone (n = 6-8) following a cumulative dose-response protocol. Different dose ratio combinations of NSAIDs and gabapentin or pregabalin (n=6-8) were tested as single, non-cumulative administration.

Drug Preparation and Volume of Administration. Gabapentin, naproxen (sodium salt), and diclofenac (sodium salt) were dissolved in distilled water. Nimesulide, flurbiprofen and sulindac were dissolved in distilled water containing DMF (25% v/v) and Tween 80 (8% v/v). Dosages were administered by injection in a volume of 1 ml per kg body weight.

### Experimental design:

a) Saline was continuously infused at a rate of 0.055 ml/min by mean of a peristaltic pump via the bladder-filling catheter for 60 minutes to obtain a baseline of bladder activity. Then the infusion pump was stopped and the bladder was emptied of residual fluid by capillarity via the infusion catheter. After this procedure, a single filling cystometrogram was performed in order to determine the two urodynamic parameters: bladder volume capacity (BVC) and micturition pressure (MP) (BVC and MP BASAL VALUES).

b) Afterwards, a 0.25% acetic acid solution in saline was infused into the bladder at the same flow rate to induce bladder irritation. After 30 minutes of acetic acid infusion, 3 vehicle injections were made at 20 minute intervals. Then the infusion pump was stopped, the bladder was emptied and a single filling cystometrogram was recorded as reported before (BVC and MP VALUES AFTER IRRITATION).

c) Subsequently, a single dose of gabapentin, pregabalin or NSAID was administered intravenously and after 20 min the infusion pump was stopped, the bladder was emptied and a single filling cystometrogram was obtained as reported before (BVC and MP VALUES AFTER TREATMENT). This procedure was repeated for three times in order to follow a cumulative dose-response protocol.

For the combination of gabapentin + NSAID and pregabalin + NSAID only one single intravenous administration/rat was made under the same conditions.

### Data Analysis

BVC is defined as the volume (mL) of fluid (saline or dilute acetic acid) infused in the bladder necessary to induce detrusor contractions followed by micturition. MP is defined as the maximal intravesical pressure (mm Hg) determined by the contraction of the detrusor during micturition.

Only rats that showed a reduction in bladder capacity between 50% and 90% after acetic acid infusion were utilized.

Changes in bladder capacity for each animal were expressed as " % bladder volume capacity vs. basal (saline infusion)" and an increase in this index was used as measure of efficacy (recovery from irritation to basal value). Data from experiments in which each drug was administered alone were utilized to calculate theoretical curves of additive effects for each fixed-ratio combination. The calculated additive curve was then compared to the experimental combination curve by 2-Way ANOVA in order to evaluate synergic or additive effect by comparing the equieffective dose, e.g., ED₆₀ for the recovery of bladder capacity to basal value, for the two curves as described in R. Tallarida, "Drug synergism and dose-effect data analysis," 57-87 (2000) Chapman & Hall/CRC Press, Boca Raton FL.

### Results

Results are given in Tables 1-16 and Figures 1A-15. Infusion of the bladder with acetic acid induced a significant reduction of BVC. Most combinations tested exhibited synergistic (i.e., superadditive) activity in increasing the bladder volume capacity in this animal model of overactive bladder.

The basal MP values (during saline infusion) resulted in the normal range, (20-40 mmHg) and variable. The infusion with acetic acid generally induced an increase of MP that recovered to basal values after treatment with gabapentin, pregabalin or anti-inflammatory drugs (data not shown).

**Table 1**

| **% Bladder Volume Capacity (vs basal) ± S.E.** | | | | | | | | | |
|---|---|---|---|---|---|---|---|---|---|
| **Compound** | **Dose (mg/kg) i.v.** | | | | | | | | |
| | **0.1** | **0.3** | **1.0** | **3** | **10** | **30** | **60** | **100** | **300** |
| Diclofenac | | 41±10 | 63±13 | 82±15 | | | | | |
| Flurbiprofen | 43±11 | 42±6 | 49±9 | 62±10 | 73±12 | | | | |
| Naproxen | | 29±8 | 36±8 | 41±7 | 48±11 | 60±12 | | | |
| Nimesulide | | 38±10 | 36±12 | 37±7 | 47±15 | | | | |
| Sulindac | | 27±5 | 39±11 | 34±8 | 38±4 | 54±8 | | | |
| Gabapentin | | | | | | 27±4 | 51±15 | 55±9 | 82±12 |
| Pregabalin | | | | | 31±6 | 35±6 | | 52±8 | |
| | **1^{st} adm.** | **2^{nd} adm.** | **3^{rd} adm.** | | | | | | |
| Vehicle | 32±2 | 31±3 | 36±4 | | | | | | |

| | | | | | | | | | |
|---|---|---|---|---|---|---|---|---|---|
| Single drugs were administered in a cumulative way. | | | | | | | | | |

**Table 2**

| **% Bladder Volume Capacity (vs basal) ± S.E.** | | | | | | | | | |
|---|---|---|---|---|---|---|---|---|---|
| **Compound** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Naproxen | | 29±8 | 36±8 | 41±7 | 48±11 | 60±12 | | | |
| Gabapentin | | | | | | 27±4 | 51±15 | 55±9 | 82±12 |
| | **3+10** | | | **10+30** | | | | | |
| Naproxen: Gabapentin Combo (1:3) | 47±6 | **Additive effect** | | 72±6 | **Synergism assumption** | | | | |

| | | | | | | | | | |
|---|---|---|---|---|---|---|---|---|---|
| Single drugs were administered in a cumulative way. The combination doses were single administrations. | | | | | | | | | |

**Table 3**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Naproxen | | 29±8 | 36±8 | 41±7 | 48±11 | 60±12 | | | |
| Gabapentin | | | | | | 27±4 | 51±15 | 55±9 | 82±12 |
| | **1+10** | **3+30** | **10+100** | | | | | | |
| Naproxen: Gabapentin Combo(1:10) | 51±11 | 83±16 | 124±21 | **Synergism (p<0.01)** | | | | | |

| | | | | | | | | | |
|---|---|---|---|---|---|---|---|---|---|
| Single drugs were administered in a cumulative way. The combination doses were single administrations. | | | | | | | | | |

**Table 4**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Naproxen | | 29±8 | 36±8 | 41±7 | 48±11 | 60±12 | | | |
| Gabapentin | | | | | | 27±4 | 51±15 | 55±9 | 82±12 |
| | **1+30** | | | | | | | | |
| Naproxen: Gabapentin Combo (1:30) | 73±17 | **Synergism assumption** | | | | | | | |

| | | | | | | | | | |
|---|---|---|---|---|---|---|---|---|---|
| Single drugs were administered in a cumulative way. The combination doses were single administrations. | | | | | | | | | |

**Table 5**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Naproxen | | 29±8 | 36±8 | 41±7 | 48±11 | 60±12 | | | |
| Gabapentin | | | | | | 27±4 | 51±15 | 55±9 | 82±12 |
| | **1+60** | | | | | | | | |
| Naproxen: Gabapentin Combo (1:60) | 73±10 | **Synergism assumption** | | | | | | | |

| | | | | | | | | | |
|---|---|---|---|---|---|---|---|---|---|
| Single drugs were administered in a cumulative way. The combination doses were single administrations. | | | | | | | | | |

**Table 6**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Naproxen | | 29±8 | 36±8 | 41±7 | 48±11 | 60±12 | | | |
| Gabapentin | | | | | | 27±4 | 51±15 | 55±9 | 82±12 |
| | **0.1+10** | **0.3+30** | **1+100** | | | | | | |
| Naproxen: Gabapentin Combo (1:100) | 27±5 | 57±10 | 79±11 | **Synergism (p<0.01)** | | | | | |

| | | | | | | | | | |
|---|---|---|---|---|---|---|---|---|---|
| Single drugs were administered in a cumulative way. The combination doses were single administrations. | | | | | | | | | |

**Table 7**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Flurbiprofen | 43±11 | 42±6 | 49±9 | 62±10 | 73±12 | | | | |
| Gabapentin | | | | | | 27±4 | 51±15 | 55±9 | 82±12 |
| | **0.03+3** | **0.1+10** | **0.3+30** | | | | | | |
| Flurbiprofen: Gabapentin Combo (1:100) | 28±7 | 44±6 | 117±23 | **Synergism (p<0.01)** | | | | | |

| | | | | | | | | | |
|---|---|---|---|---|---|---|---|---|---|
| Single drugs were administered in a cumulative way. The combination doses were single administrations. | | | | | | | | | |

**Table 8**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Nimesulide | | 38±10 | 36±12 | 37±7 | 47±15 | | | | |
| Gabapentin | | | | | | 27±4 | 51±15 | 55±9 | 82±12 |
| | **10+30** | | | | | | | | |
| Nimesulide: Gabapentin Combo (1:3) | 64±11 | **Synergism assumption** | | | | | | | |

| | | | | | | | | | |
|---|---|---|---|---|---|---|---|---|---|
| Single drugs were administered in a cumulative way. The combination doses were single administrations. | | | | | | | | | |

**Table 9**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Nimesulide | | 38±10 | 36±12 | 37±7 | 47±15 | | | | |
| Gabapentin | | | | | | 27±4 | 51±15 | 55±9 | 82±12 |
| | **1+10** | **3+30** | **10+100** | | | | | | |
| Nimesulide: Gabapentin Combo (1:10) | 69±10 | 73±8 | 134±24 | **Synergism (p<0.01)** | | | | | |

| | | | | | | | | | |
|---|---|---|---|---|---|---|---|---|---|
| Single drugs were administered in a cumulative way. The combination doses were single administrations. | | | | | | | | | |

**Table 10**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Diclofenac | | 41±10 | 63±13 | 82±15 | | | | | |
| Gabapentin | | | | | | 27±4 | 51±15 | 55±9 | 82±12 |
| | **0.1+1** | **0.3+3** | **1+10** | | | | | | |
| Diclofenac: Gabapentin Combo (1:10) | 38±8 | 50±10 | 77±12 | **Synergism (P<0.01)** | | | | | |

| | | | | | | | | | |
|---|---|---|---|---|---|---|---|---|---|
| Single drugs were administered in a cumulative way. The combination doses were single administrations. | | | | | | | | | |

**Table 11**

| **% Bladder Volume Capacity (vs basal) ± S.E.** | | | | | | | | | |
|---|---|---|---|---|---|---|---|---|---|
| **Compounds** | **Dose (mg/kg) i.v.** | | | | | | | | |
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Flurbiprofen | 43±11 | 42±6 | 49±9 | 62±1- | 73±12 | | | | |
| Gabapentin | | | | | | 27±4 | 51±15 | 55±9 | 82±12 |
| | **0.1+1** | **0.3+3** | **1+10** | **3+30** | | | | | |
| Flurbiprofen: Gabapentin Combo (1:10) | 46±6 | 70±12 | 74±14 | 92±16 | **Synergism (p<0.01)** | | | | |

| | | | | | | | | | |
|---|---|---|---|---|---|---|---|---|---|
| Single drugs were administered in a cumulative way. The combination doses were single administrations. | | | | | | | | | |

**Table 12**

| **% Bladder Volume Capacity (vs basal) ± S.E.** | | | | | | | | | |
|---|---|---|---|---|---|---|---|---|---|
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Sulindac | | 27±5 | 39±11 | 34±8 | 38±4 | 54±8 | | | |
| Gabapentin | | | | | | 27±4 | 51±15 | 55±9 | 82±12 |
| | **1+10** | **3+30** | **10+100** | | | | | | |
| Sulindac: Gabapentin Combo (1:10) | 41±7 | 69±10 | 116±19 | **Synergism (p<0.05)** | | | | | |

| | | | | | | | | | |
|---|---|---|---|---|---|---|---|---|---|
| Single drugs were administered in a cumulative way. The combination doses were single administrations. | | | | | | | | | |

**Table 13**

| **% Bladder Volume Capacity (vs basal) ± S.E.** | | | | | | | | | |
|---|---|---|---|---|---|---|---|---|---|
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Flurbiprofen | 43±11 | 42±6 | 49±9 | 62±10 | 73±12 | | | | |
| Gabapentin | | | | | | 27±4 | 51±15 | 55±9 | 82±12 |
| | **0.13+3.3** | **0.39+10** | **1.17+30** | **3.5+90** | | | | | |
| Flurbiprofen: Gabapentin Combo (1:26) | 45±5 | 54±9 | 80±14 | 117±18 | **Synergism (p<0.05)** | | | | |

| | | | | | | | | | |
|---|---|---|---|---|---|---|---|---|---|
| Single drugs were administered in a cumulative way. The combination doses were single administrations. | | | | | | | | | |

**Table 14**

| **% Bladder Volume Capacity (vs basal) ± S.E.** | | | | | | | | | |
|---|---|---|---|---|---|---|---|---|---|
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Flurbiprofen | 43±11 | 42±6 | 49±9 | 62±10 | 73±12 | | | | |
| Gabapentin | | | | | | 27±4 | 51±15 | 55±9 | 82±12 |
| | **0.058+4.63** | **0.115+9.25** | **0.23+18.5** | **0.46±37** | | | | | |
| Flurbiprofen: Gabapentin Combo (1:80) | 47±8 | 66±10 | 91±10 | 118±25 | **Synergism (p<0.05)** | | | | |

| | | | | | | | | | |
|---|---|---|---|---|---|---|---|---|---|
| Single drugs were administered in a cumulative way. The combination doses were single administrations. | | | | | | | | | |

**Table 15**

| **% Bladder Volume Capacity (vs basal) ± S.E.** | | | | | | | | | |
|---|---|---|---|---|---|---|---|---|---|
| | **1^{st} adm.** | **2^{nd} adm.** | **3^{rd} adm.** | | | | | | |
| Vehicle | 32±2 | 31±3 | 36±4 | | | | | | |
| | **0.1** | **0.3** | **1.0** | **3** | **10** | **30** | **60** | **100** | **300** |
| Flurbiprofen | 43±11 | 42±6 | 49±9 | 62±10 | 73±12 | | | | |
| Pregabalin | | | | | 31±6 | 35±6 | | 52±8 | |
| | **0.3+3** | **1+10** | **3+30** | | | | | | |
| Flurbiprofen: Pregabalin Combo (1:10) | 62±10 | 88±12 | 108±9 | **Synergism (p<0.05)** | | | | | |

| | | | | | | | | | |
|---|---|---|---|---|---|---|---|---|---|
| Single drugs were administered in a cumulative way. The combination doses were single administrations. | | | | | | | | | |

**Table 16**

| **ED₆₀ of recovery of Bladder Volume Capacity to basal value** | | | |
|---|---|---|---|
| **COMPOUND** | **ED₆₀ (CL 95%)** | **Ratio ED₆₀ Additive/ ED₆₀ Experimental** | **ANOVA** |
| Flurbiprofen | 2.34 (nc-4.77) | | |
| Naproxen | 44.7 (0.8-88.6) | | |
| Diclofenac | 0.85 (0.80-0.90) | | |
| Nimesulide | >>10 | | |
| Sulindac | >30 | | |
| Gabapentin | 112 (75-149) | | |
| Pregabalin | 300.5 (n.c.-n.c.) | | |
| Naproxen + Gabapentin 1:10 | | 8.15 | P<0.01 |
| Calculated Additive | 122.2 (79.9-154.5) | | |
| Experimental | 15.0 (11.9-18.1) | | |
| Naproxen + Gabapentin 1:100 | | 2.97 | P<0.01 |
| Calculated Additive | 120.8 (107.9-133.7) | | |
| Experimental | 40.6 (22.5-58.7) | | |
| Flurbiprofen + Gabapentin 1:10 | | 7.85 | P<0.01 |
| Calculated Additive | 20.4 (18.8-22.0) | | |
| Experimental | 2.60 (0.22-5.00) | | |
| Flurbiprofen + Gabapentin | | 8.24 | P<0.01 |
| 1:100 | 75.0 (61.9-88.1) | | |
| Calculated Additive Experimental | 9.1 (nc-24.1) | | |
| Diclofenac + Gabapentin 1:10 | | 1.58 | P<0.01 |
| Calculated Additive | 8.04 (7.93-8.15). | | |
| Experimental | 5.08 (4.08-6.08) | | |
| Nimesulide + Gabapentin 1:10 | | 11.3 | P<0.01 |
| Calculated Additive | 124.4 (82.7-166.1) | | |
| Experimental | 11.0 (1.2-21.0) | | |
| Sulindac + Gabapentin 1:10 | | 5.86 | P<0.05 |
| Calculated Additive | 125.4 (92.8-157.3) | | |
| Experimental | 21.4 (8.9-34.0) | | |
| Flurbiprofen + Gabapentin 1:26 | | 3.33 | P<0.05 |
| Calculated Additive | 31.6 (16.6-46.6) | | |
| Experimental | 9.5 (1.9-17.0) | | |
| Flurbiprofen + Gabapentin 1:80 | | 8.14 | P<0.05 |
| Calculated Additive | 59.4 (28.2-90.6) | | |
| Experimental | 7.3 (6.2-8.5) | | |
| Flurbiprofen + Pregabalin 1:10 | | 7.55 | P<0.05 |
| Calculated Additive | 21.9 (7.4-36.3) | | |
| Experimental | 2.9 (2.1-3.6) | | |

## Claims

1. An α₂δ calcium channel subunit (A2d) ligand selected from gabapentin, pregabalin, (1R,5R,6S)-6-aminomethyl-6-carboxymethyl-bicyclo[3.2.0]heptane, 3-(1-aminomethylcyclohexylmethyl)-4H-1,2,4-oxadiazol-5-one, 5-(1-aminomethyl-cyclohexylmethyl)-1H-tetrazole, (3S,4S)-(1-aminomethyl-1-carboxymethyl-3,4-dimethyl-cyclopentane, (1α,3α,5α)-(3-aminomethyl-3-carboxymethyl-bicyclo[3.2.0]heptane, (3S,5R)-3-aminomethyl-5-methyloctanoic acid, (3S,5R)-3-amino-5-methyl-heptanoic acid, (3S,5R)-3-amino-5-methyloctanoic acid, (3S,5R)-3-amino-5-methyl-nonanoic acid, (2S,4S)-4-(3-chlorophenoxy)-proline or (2S,4S)-4-(3-fluorobenzyl)-proline, or a mixture of two or more thereof, for use in the treatment of urinary incontinence in a subject suffering from a lower urinary tract disorder in combination with the prior, concurrent or post-administration of a non-steroidal anti-inflammatory drug (NSAID).

2. A non-steroidal anti-inflammatory drug (NSAID) for use in the treatment of urinary incontinence in a subject suffering from a lower urinary tract disorder in combination with the prior, concurrent or post-administration of an α₂δ calcium channel subunit (A2d) ligand selected from gabapentin, pregabalin, (1R,5R,6S)-6-aminomethyl-6-carboxymethyl-bicyclo[3.2.0]heptane, 3-(1-aminomethyl-cyclohexylmethyl)-4H-1,2,4-oxadiazol-5-one, 5-(1-aminomethyl-cyclohexylmethyl)- 1H-tetrazole, (3S,4S)-(1-aminomethyl-1-carboxymethyl-3,4-dimethyl-cyclopentane, (1α,3α,5α)-(3-aminomethyl-3-carboxymethyl-bicyclo[3.2.0]heptane, (3S,5R)-3-aminomethyl-5-methyl-octanoic acid, (3S,5R)-3-amino-5-methyl-heptanoic acid, (3S,5R)-3-amino-5-methyl-octanoic acid, (3S,5R)-3-amino-5-methyl-nonanoic acid, (2S,4S)-4-(3-chlorophenoxy)-proline or (2S,4S)-4-(3-fluorobenzyl)-proline, or a mixture of two or more thereof.

3. An NSAID for use according to claim 2 which is a COX-inhibitor.

4. An NSAID for use according to claim 2 or claim 3 which is celecoxib, diclofenac, diflunisal, flurbiprofen, naproxen, nimesulide or sulindac.

5. An α₂δ calcium channel subunit (A2d) ligand selected from gabapentin, pregabalin, (1R,SR,6S)-6-aminomethyl-6-carboxymethyl-bicyclo[3.2.0]heptane, 3-(1-aminomethyl-cyclohexylmethyl)-4H-1,2,4-oxadiazol-5-one, 5-(1-aminomethyl-cyclohexylmethyl)-1H-tetrazole, (3S,4S)-(1-aminomethyl-1-carboxymethyl-3,4-dimethyl-cyclopentane, (1α,3α,5α)-(3-aminomethyl-3-carboxymethyl-bicyclo[3.2.0]heptane, (3S,5R)-3-aminomethyl-5-methyl-octanoic acid, (3S,5R)-3-amino-5-methyl-heptanoic acid, (3S,5R)-3-amino-5-methyl-octanoic acid, (3S,5R)-3-amino-5-methyl-nonanoic acid, (2S,4S)-4-(3-chlorophenoxy)-proline or (2S,4S)-4-(3-fluorobenzyl)-proline, or a mixture of two or more thereof, and a non-steroidal anti-inflammatory drug (NSAID) for use concurrently or sequentially in the treatment of urinary incontinence in a subject suffering from a lower urinary tract disorder.

6. An A2d ligand and NSAID for use according to claim 5 in which the NSAID is a COX-inhibitor.

7. An A2d ligand and NSAID for use according to claim 5 or claim 6 in which the NSAID is celecoxib, diclofenac, diflunisal, flurbiprofen, naproxen, nimesulide or sulindac.

8. Use of an α₂δ calcium channel subunit (A2d) ligand selected from gabapentin, pregabalin, (1R,5R,6S)-6-aminomethyl-6-carboxymethyl-bicyclo[3.2.0]heptane, 3-(1-aminomethyl-cyclohexylmethyl)-4H-1,2,4-oxadiazol-5-one, 5-(1-aminomethyl-cyclohexylmethyl)-1H-tetrazole, (3S,4S)-(1-aminomethyl-1-carboxymethyl-3,4-dimethyl-cyclopentane, (1α,3α,5α)-(3-aminomethyl-3-carboxymethyl-bicyclo[3.2.0]heptane, (3S,5R)-3-aminomethyl-5-methyl-octanoic acid, (3S,5R)-3-amino-5-methyl-heptanoic acid, (3S,5R)-3-amino-5-methyl-octanoic acid, (3S,5R)-3-amino-5-methyl-nonanoic acid, (2S,4S)-4-(3-chlorophenoxy)-proline or (2S,4S)-4-(3-fluorobenzyl)-proline, or a mixture of two or more thereof, for the preparation of a medicament for the treatment of urinary incontinence in a subject suffering from a lower urinary tract disorder in combination with the prior, concurrent or post-administration of a non-steroidal anti-inflammatory drug (NSAID).

9. Use of a non-steroidal anti-inflammatory drug (NSAID) for the preparation of a medicament for the treatment of urinary incontinence in a subject suffering from a lower urinary tract disorder in combination with the prior, concurrent or post-administration of an α₂δ calcium channel subunit (A2d) ligand selected from gabapentin, pregabalin, (1R,5R,6S)-6-aminomethyl-6-carboxymethyl-bicyclo[3.2.0]heptane, 3-(1-aminomethyl-cyclohexylmethyl)-4H-1,2,4-oxadiazol-5-one, 5-(1-aminomethyl-cyclohexylmethyl)-1H-tetrazole, (3S,4S)-(1-aminomethyl-1-carboxymethyl-3,4-dimethyl-cyclopentane, (1α,3α,5α)-(3-aminomethyl-3-carboxymethyl-bicyclo[3.2.0]heptane, (3S,SR)-3-aminomethyl-5-methyl-octanoic acid, (3S,5R)-3-amino-5-methyl-heptanoic acid, (3S,5R)-3-amino-5-methyl-octanoic acid, (3S,5R)-3-amino-5-methyl-nonanoic acid, (2S,4S)-4-(3-chlorophenoxy)-proline or (2S,4S)-4-(3-fluorobenzyl)-proline, or a mixture of two or more thereof.

10. Use according to claim 8 or claim 9 in which the NSAID is a COX-inhibitor.

11. Use according to claim 10 in which the NSAID is celecoxib, diclofenac, diflunisal, flurbiprofen, naproxen, nimesulide or sulindac.

## Patentansprüche

1. α₂δ Kalziumkanaluntereinheit (A2d) Ligand, ausgewählt aus Gabapentin, Pregabalin, (1R,5R,6S)-6-aminomethyl-6-carboxymethyl-bicyclo[3.2.0]heptan, 3-(1-aminomethyl-cyclohexylmethyl)-4H-1,2,4-oxadiazol-5-on, 5-(1-aminomethyl-cyclohexylmethyl)-1H-tetrazol, (3S,4S)-(1-aminomethyl-1-carboxymethyl-3,4-dimethyl-cyclopentan, (1α,3α,5α)-(3-aminomethyl-3-carboxymethyl-bicyclo[3.2.0]heptan, (3S,5R)-3-aminomethyl-5-methyl-Oktansäure, (3S,5R)-3-amino-5-methyl-heptansäure, (3S,5R)-3-amino-5-methyl-oktansäure, (3S,5R)-3-amino-5-methyl-nonansäure, (2S,4S)-4-(3-chlorophenoxy)-prolin oder (2S,4S)-4-(3-fluorobenzyl)-prolin oder ein Gemisch von 2 oder mehreren davon, zur Behandlung von Harninkontinenz in einem Probanden, der unter einer Funktionsstörung des unteren Harntrakts leidet, in Kombination mit der vorherigen, gleichzeitigen oder anschließenden Verabreichung eines nicht-steroidalen Antirheumatikums (NSAR).

2. Nicht-steroidales Antirheumatikum (NSAR) zur Behandlung von Harninkontinenz in einem Probanden, der an einer Funktionsstörung des unteren Harntraktes leidet, in Kombination mit der vorherigen, gleichzeitigen oder anschließenden Verabreichung eines α₂δ Kalziumkanaluntereinheit (A2d) Liganden, ausgewählt aus Gabapentin, Pregabalin, (1R,5R,6S)-6-aminomethyl-6-carboxymethyl-bicyclo[3.2.0]heptan, 3-(1-aminomethyl-cyclohexylmethyl)-4H-1,2,4-oxadiazol-5-on, 5-(1-aminomethyl-cyclohexylmethyl)-1H-tetrazol, (3S,4S)-(1-aminomethyl-1-carboxymethyl-3,4-dimethyl-cyclopentan, (1α,3α,5α)-(3-aminomethyl-3-carboxymethyl-bicyclo[3.2.0]heptan, (3S,5R)-3-aminomethyl-5-methyl-Oktansäure, (3S,5R)-3-amino-5-methyl-heptansäure, (3S,5R)-3-amino-5-methyl-oktansäure, (3S,5R)-3-amino-5-methyl-nonansäure, (2S,4S)-4-(3-chlorophenoxy)-prolin oder (2S,4S)-4-(3-fluorobenzyl)-prolin oder ein Gemisch von 2 oder mehreren davon.

3. NSAR zur Verwendung gemäß Anspruch 2, welches ein COX-Inhibitor ist.

4. NSAR zur Verwendung gemäß Anspruch 2 oder 3, welches Celecoxib, Diclofenac, Diflunisal, Flurbiprofen, Naproxen, Nimesulfid oder Sulindac ist.

5. α₂δ Kalziumkanaluntereinheit (A2d)-Ligand, ausgewählt aus Gabapentin, Pregabalin, (1R,5R,6S)-6-aminomethyl-6-carboxymethyl-bicyclo[3.2.0]heptan, 3-(1-aminomethyl-cyclohexylmethyl)-4H-1,2,4-oxadiazol-5-on, 5-(1-aminomethyl-cyclohexylmethyl)-1H-tetrazol, (3S,4S)-(1-aminomethyl-1-carboxymethyl-3,4-dimethyl-cyclopentan, (1α,3α,5α)-(3-aminomethyl-3-carboxymethyl-bicyclo[3.2.0]heptan, (3S,5R)-3-aminomethyl-5-methyl-Oktansäure, (3S,SR)-3-amino-5-methyl-heptansäure, (3S,5R)-3-amino-5-methyl-oktansäure, (3S,5R)-3-amino-5-methyl-nonansäure, (2S,4S)-4-(3-chlorophenoxy)-prolin oder (2S,4S)-4-(3-fluorobenzyl)-prolin oder ein Gemisch von 2 oder mehreren davon und ein nicht-steroidales Antirheumatikum (NSAR) zur gleichzeitigen oder sequentiellen Verwendung bei der Behandlung von Harninkontinenz in einem Probanden, der an einer Funktionsstörung des unteren Harntrakts leidet.

6. Ein A2d Ligand und NSAR zur Verwendung gemäß Anspruch 5, wobei das NSAR ein Cox-Inhibitor ist.

7. Ein A2d-Ligand und NSAR zur Verwendung gemäß Anspruch 5 oder Anspruch 6, wobei das NSAR Celecoxib, Diclofenac, Diflunisal, Flurbiprofen, Naproxen, Nimesulfid oder Sulindac ist.

8. Verwendung eines α₂δ-Kalziumkanaluntereinheit (A2d) Liganden, ausgewählt aus Gabapentin, Pregabalin, (1R,5R,6S)-6-aminomethyl-6-carboxymethyl-bicyclo[3.2.0]heptan, 3-(1-aminomethyl-cyclohexylmethyl)-4H-1,2,4-oxadiazol-5-on, 5-(1-aminomethyl-cyclohexymethyl)-1H-tetrazol, (3S,4S)-(1-aminomethyl-1-carboxymethyl-3,4-dimethyl-cyclopentan, (1α,3α,Sα)-(3-aminomethyl-3-carboxymethyl-bicyclo[3.2.0]heptan, (3S,5R)-3-aminomethyl-5-methyl-Oktansäure, (3S,5R)-3-amino-5-methyl-heptansäure, (3S,5R)-3-amino-5-methyl-oktansäure, (3S,5R)-3-amino-5-methyl-nonansäure, (2S,4S)-4-(3-chlorophenoxy)-prolin oder (2S,4S)-4-(3-fluorobenzyl)-prolin oder ein Gemisch von 2 oder mehreren davon, für die Herstellung eines Medikaments zur Behandlung von Harninkontinenz in einem Probanden, der an einer Funktionsstörung des unteren Harntrakts leidet, in Kombination mit der vorherigen, gleichzeitigen oder anschließenden Verabreichung eines nicht-steroidalen Antirheumatikums (NSAR).

9. Verwendung eines nicht-steroidalen Antirheumatikums (NSAR) zur Herstellung eines Medikaments zur Behandlung von Harninkontinenz in einem Probanden, der an einer Funktionsstörung des unteren Harntrakts leidet in Kombination mit der vorherigen, gleichzeitigen oder anschließenden Verabreichung eines α₂δ-Kalziumkanaluntereinheit (A2d)-Liganden, ausgewählt aus Gabapentin, Pregabalin, (1R,%R,6S)-6-aminomethyl-6-carboxymethyl-bicyclo[3.2.0]heptan, 3-(1-aminomethyl-cyclohexymethyl)-4H-1,2,4-oxadiazol-5-on, 5-(1-aminomethyl-cyclohexylmethyl)-1H-tetrazol, (3S,4S)-(1-aminomethyl-1-carboxymethyl-3,4-dimethyl-cyclopentan, (1α,3α,Sα)-(3-aminomethyl-3-carboxymethyl-bicyclo[3.2.0]heptan, (3S,SR)-3-aminomethyl-5-methyl-Oktansäure, (3S,5R)-3-amino-5-methyl-heptansäure, (3S,5R)-3-amino-5-methyl-oktansäure, (3S,5R)-3-amino-5-methyl-nonansäure, (2S,4S)-4-(3-chlorophenoxy)-prolin oder (2S,4S)-4-(3-fluorobenzyl)-prolin oder ein Gemisch von 2 oder mehreren davon.

10. Verwendung gemäß Anspruch 8 oder Anspruch 9, wobei das NSAR ein COX-Inhibitor ist.

11. Verwendung gemäß Anspruch 10, wobei das NSAR Celecoxib, Declofenac, Diflunisal, Flurbiprofen, Naproxen, Nimesulfid oder Sulindac ist.

## Revendications

1. Ligand de la sous-unité de canal calcique α₂δ (A2d) choisi parmi la gabapentine, la prégabaline, (1R,5R,6S)-6-aminométhyl-6-carboxyméthyl-bicyclo[3.2.0]heptane, 3-(1-aminométhyl-cyclohexylméthyl)-4H-1,2,4-oxadiazol-5-one, 5-(1-aminométhyl-cyclohexylméthyl)-1H-tétrazole, (3S,4S)-(1-aminométhyl-1-carboxyméthyl-3,4-diméthyl-cyclopentane, (1α,3α,5α)-(3-amino-méthyl-3-carboxyméthyl-bicyclo[3.2.0]heptane, l'acide (3S,5R)-3-aminométhyl-5-méthyl-octanoïque, l'acide (3S,5R)-3-amino-5-méthyl-heptanoïque, l'acide (3S,5R)-3-amino-5-méthyl-octanoïque, l'acide (3S,5R)-3-amino-5-méthyl-nonanoïque, (2S,4S)-4-(3-chlorophénoxy)-proline ou (2S,4S)-4-(3-fluorobenzyl)-proline, ou un mélange de deux ou plusieurs de ceux-ci, pour utilisation dans le traitement de l'incontinence urinaire chez un sujet souffrant d'un trouble du tractus urinaire inférieur en combinaison avec l'administration préalable, simultanée ou postérieure d'un médicament anti-inflammatoire non stéroïdien (AINS).

2. Médicament anti-inflammatoire non stéroïdien (AINS) pour utilisation dans le traitement de l'incontinence urinaire chez un sujet souffrant d'un trouble du tractus urinaire inférieur en combinaison avec l'administration préalable, simultanée ou postérieure d'un ligand de la sous-unité de canal calcique α₂δ (A2d) choisi parmi la gabapentine, la prégabaline, (1R,5R,6S)-6-amino-méthyl-6-carboxyméthyl-bicyclo[3.2.0]heptane, 3-(1-aminométhyl-cyclohexylméthyl)-4H-1,2,4-oxa-diazol-5-one, 5-(1-aminométhyl-cyclohexylméthyl)-1H-tétrazole, (3S,4S)-(1-aminométhyl-1-carboxy-méthyl-3,4-diméthyl-cyclopentane, (la,3a,5a)-(3-aminométhyl-3-carboxyméthyl-bicyclo[3.2.0]heptane, l'acide (3S,5R)-3-amino-méthyl-5-méthyl-octanoïque, l'acide (3S,5R)-3-amino-5-méthyl-heptanoïque, l'acide (3S,5R)-3-amino-5-méthyl-octanoïque, l'acide (3S,5R)-3-amino-5-méthyl-nonanoïque, (2S,4S)-4-(3-chlorophénoxy)-proline ou (2S,4S)-4-(3-fluorobenzyl)-proline, ou un mélange de deux ou plusieurs de ceux-ci.

3. AINS pour son utilisation selon la revendication 2 qui est un inhibiteur de COX.

4. AINS pour son utilisation selon la revendication 2 ou la revendication 3 qui est le célécoxib, le diclofénac, le diflunisal, le flurbiprofène, le naproxène, le nimésulide, ou le sulindac.

5. Ligand de la sous-unité de canal calcique α₂δ (A2d) choisi parmi la gabapentine, la prégabaline, (1R,5R,6S)-6-aminométhyl-6-carboxyméthyl-bicyclo[3.2.0]heptane, 3-(1-aminométhyl-cyclohexylméthyl)-4H-1,2,4-oxadiazol-5-one, 5-(1-aminométhyl-cyclohexylméthyl)-1H-tétrazole, (3S,4S)-(1-aminométhyl-1-carboxyméthyl-3,4-diméthyl-cyclopentane, (1α,3α,5α)-(3-amino-méthyl-3-carboxyméthyl-bicyclo[3.2.0]heptane, l'acide (3S,5R)-3-aminométhyl-5-méthyl-octanoïque, l'acide (3S,5R)-3-amino-5-méthyl-heptanoïque, l'acide (3S,5R)-3-amino-5-méthyl-octanoïque, l'acide (3S,5R)-3-amino-5-méthyl-nonanoïque, (2S,4S)-4-(3-chlorophénoxy)-proline ou (2S,4S)-4-(3-fluorobenzyl)-proline, ou un mélange de deux ou plusieurs de ceux-ci, et un médicament anti-inflammatoire non-stéroïdien (AINS) pour son utilisation simultanée ou consécutive dans le traitement de l'incontinence urinaire chez un sujet souffrant d'un trouble du tractus urinaire inférieur.

6. Ligand de A2d et AINS pour leur utilisation selon la revendication 5 dans laquelle l'AINS est un inhibiteur de COX.

7. Ligand de A2d et AINS pour leur utilisation selon la revendication 5 ou la revendication 6 dans laquelle l'AINS est le célécoxib, le diclofénac, le diflunisal, le flurbiprofène, le naproxène, le nimésulide ou le sulindac.

8. Utilisation d'un ligand de sous-unité de canal calcique α₂δ (A2d) choisi parmi la gabapentine, la prégabaline, (1R,5R,6S)-6-aminométhyl-6-carboxy-méthyl-bicyclo[3.2.0]heptane, 3-(1-aminométhyl-cyclohexylméthyl)-4H-1,2,4-oxadiazol-5-one, 5-(1-aminométhyl-cyclohexylméthyl)-1H-tétrazole, (3S,4S)-(1-aminométhyl-1-carboxyméthyl-3,4-diméthyl-cyclopentane, (1α,3α,5α)-(3-amino-méthyl-3-carboxyméthyl-bicyclo[3.2.0]heptane, l'acide (3S,5R)-3-aminométhyl-5-methyl-octanoïque,
l'acide (3S,5R)-3-amino-5-méthyl-heptanoïque, l'acide (3S,5R)-3-amino-5-méthyl-octanoïque, l'acide (3S,5R)-3-amino-5-méthyl-nonanoïque, (2S,4S)-4-(3-chlorophénoxy)-proline ou (2S,4S)-4-(3-fluorobenzyl)-proline, ou un mélange de deux ou plusieurs de ceux-ci, pour la préparation d'un médicament destiné au traitement de l'incontinence urinaire chez un patient souffrant d'un trouble du tractus urinaire inférieur en combinaison avec l'administration préalable, simultanée ou postérieure d'un médicament anti-inflammatoire non stéroïdien (AINS).

9. Utilisation d'un médicament anti-inflammatoire non stéroïdien (AINS) pour la préparation d'un médicament destiné au traitement de l'incontinence urinaire chez un sujet souffrant d'un trouble du tractus urinaire inférieur en combinaison avec l'administration préalable, simultanée ou postérieure d'un ligand (A2d) d'une sous-unité de canal calcique α2δ choisi parmi la gabapentine, la prégabaline, (1R,5R,6S)-6-aminométhyl-6-carboxy-méthyl-bicyclo[3.2.0]heptane, 3-(1-aminométhyl-cyclohexylméthyl)-4H-1,2,4-oxadiazol-5-one, 5-(1-aminométhyl-cyclohexylméthyl)-1H-tétrazole, (3S,4S)-(1-aminométhyl-1-carboxyméthyl-3,4-diméthyl-cyclopentane, (1α,3α,5α)-(3-amino-méthyl-3-carboxyméthyl-bicyclo[3.2.0]heptane, l'acide (3S,5R)-3-aminométhyl-5-méthyl-octanoïque, l'acide (3S,5R)-3-amino-5-méthyl-heptanoïque, l'acide (3S,5R)-3-amino-5-méthyl-octanoïque, l'acide (3S,5R)-3-amino-5-méthyl-nonanoïque, (2S,4S)-4-(3-chlorophénoxy)-proline ou (2S,4S)-4-(3-fluorobenzyl)-proline, ou un mélange de deux ou plusieurs de ceux-ci.

10. Utilisation selon la revendication 8 ou la revendication 9 dans laquelle l'AINS est un inhibiteur de COX.

11. Utilisation selon la revendication 10 dans laquelle l'AINS est le célécoxib, le diclofénac, le diflunisal, le flurbiprofène, le naproxène, le nimésulide, ou le sulindac.
